# EUROPÄISCHE PATENTSCHRIFT

(11) **EP 3 905 994 B1**
(45) Veröffentlichungstag und Bekanntmachung des Hinweises auf die Patenterteilung: **26.07.2023**
(21) Anmeldenummer: 19700134.0
(22) Anmeldetag: 03.01.2019
(51) Int. Cl.: A61F 5/00

(54) **GASTROINTESTINALES IMPLANTAT UND POSITIONIERVORRICHTUNG HIERFÜR**
GASTROINTESTINAL IMPLANT AND POSITIONING DEVICE FOR SAME
IMPLANT GASTRO-INTESTINAL ET DISPOSITIF DE POSITIONNEMENT POUR CELUI-CI

(43) Veröffentlichungstag der Anmeldung: 10.11.2021
(60) Teilanmeldung: 23177262.5
(73) Patentinhaber: Butz, Alexander, 1130 Wien (AT); Grablowitz, Viktor, 1010 Wien (AT)
(72) Erfinder: Butz, Alexander, 1130 Wien (AT); Grablowitz, Viktor, 1010 Wien (AT)
(74) Vertreter: KLIMENT & HENHAPEL
(86) Internationale Anmeldenummer: PCT/EP2019/050112
(87) Internationale Veröffentlichungsnummer: WO 2020/141023

(56) Entgegenhaltungen:
- WO-A1-2011/062882
- WO-A1-2012/080997
- WO-A1-2013/026473
- US-A1- 2004 107 004
- US-A1- 2005 273 060
- US-A1- 2007 010 865
- US-A1- 2008 195 226
- US-A1- 2008 255 678
- US-A1- 2012 065 571
- US-A1- 2013 030 351

## Beschreibung

### GEBIET DER ERFINDUNG

Die vorliegende Erfindung bezieht sich auf das Gebiet der Übergewichtigkeit und den damit verbundenen Folgekrankheiten wie beispielsweise Diabetes mellitus, arterielle Hypertonie und den damit einhergehenden erhöhten Schlaganfall-, Herzinfarkt und Krebsrisiken, aber auch Folgekrankheiten der Wirbelsäule und des Bewegungsapparates. US 2012/065571 A1 stellt der nächste Stand der Technik dar.

Viele Patienten sind oft nicht in der Lage, ihre Übergewichtigkeit aus eigener Kraft zu bekämpfen, beispielsweise in dem sie weniger oder gesünder essen. Oft ist es Patienten darüberhinaus auch nicht möglich, für die zur Bekämpfung der Übergewichtigkeit so notwendige Bewegung zu sorgen, da die Übergewichtigkeit oft bereits so weit fortgeschritten ist, dass die oben erwähnten Folgekrankheiten der Wirbelsäule und des Bewegungsapparates so starke Schmerzen verursachen, dass an ausreichende Bewegung nicht zu denken ist. Diese Patienten benötigend daher zusätzlich Hilfe, die es ermöglicht, das Übergewicht rasch auf ein Maß zu reduzieren, so dass zumindest schmerzfreie Bewegung wieder möglich ist. Das damit einhergehende, gesteigerte Wohlbefinden motiviert Patienten dann sehr oft aber auch, weitere Anstrengungen zur Gewichtsreduktion zu unternehmen, so dass diese initiale Hilfe zur Gewichtsreduktion den Patienten helfen, die weitere Gewichtsreduktion aus eigener Kraft zu schaffen bzw. das auf ein weniger gesundheitsschädliches Maß reduzierte Gewicht zumindest zu halten.

### STAND DER TECHNIK

Um die beschriebene rasche Gewichtsreduktion zu ermöglichen sind in der Medizin unterschiedliche Methoden bekannt. So ist es beispielsweise bekannt, Magenverkleinerungen vorzunehmen oder Magenbypasse anzulegen. Da es sich dabei um einen schwierigen, invasiven Eingriff am menschlichen Körper handelt, ist diese Methode nicht nur technisch sehr anspruchsvoll sondern auch mit einem bei jedem invasiven Eingriff einhergehenden Risiko verbunden, das unter anderem auch das mit der erforderlichen längeren Narkosezeit einhergehende Risiko umfasst.

Auf der Suche nach alternativen, weniger risikobehafteten Methoden ist man dazu übergegangen, einen Kunststoffschlauch samt Befestigungsmittel (gastrointestinales Implantat) beginnend im menschlichen Dünndarm, insbesondere Zwölffingerdarm zu positionieren und auf diese Art und Weise die Nahrungsmittelresorption teilweise auszuschalten. Die zugeführte Nahrung wird im Bereich des Kunststoffschlauchs nicht resorbiert, da der Kunststoffschlauch das Darmlumen innen auskleidet und somit den Kontakt zur Darmwand und damit die Resorption von Nährstoffen verhindert. Bei entsprechender Platzierung des Kunststoffschlauchs im Dünndarm (Zwölffingerdarm), unmittelbar im Anschluss an den Pylorus, bewirkt der Kunststoffschlauch außerdem, dass die Verdauungsenzyme (Galle- und Pankreassekret) erst nach Ende des Kunststoffschlauchs mit der Nahrung in Kontakt treten, wodurch die Nahrungsverwertung zusätzlich limitiert und der duodenogastrale Reflux verhindert wird.

Die Länge des Schlauchs kann dabei individuell gewählt werden und beeinflusst die Geschwindigkeit und das maximale Ausmaß, mit der die Gewichtsreduktion voranschreiten soll. Es sind Gewichtsreduktionen von >70% in einem Jahr möglich. Außerdem ist der Diabetes mellitus Typ II erwartungsgemäß bereits bei 80% der Patienten nach 3 Monaten in Remission, wie man in diversen Studien festgestellt hat und aus der bariatrischen Chirurgie weiß. Auch der arterielle Bluthochdruck, Herzrhythmusstörungen, Krankheiten des Bewegungsapparates und andere Komorbitäten bessern sich deutlich nach einer derartigen Gewichtsreduktion und sind sogar in Remission.

Es ist bekannt, das gastrointestinale Implantat endoskopisch in seine Position im Dünndarm zu bringen, sowie von dort auch wieder zu entfernen. Es ist somit keine Operation erforderlich. Das Verfahren ist nicht invasiv und daher narbenfrei und voll reversibel. Die Applikation des Schlauches erfolgt in Sedierung oder Kurznarkose.

Um die endoskopische Positionierung des gastrointestinalen Implantats im Dünndarm vornehmen zu können, bedarf es einerseits einer Positioniervorrichtung, welche das gastrointestinale Implantat zunächst, während des Einbringens in den Dünndarm, schützt und sodann eine Fixierung und Entfaltung des Kunststoffschlauchs ermöglicht, andererseits aber auch geeigneter Befestigungsmittel, die eine verlässliche, im Wesentlichen unverschiebbare Fixierung des Kunststoffschlauchs im Zwölffingerdarm ermöglichen.

Vor allem die Fixierung des Kunststoffschlauchs sowie dessen Entfaltung im Dünndarm bereiten dabei immer noch Probleme.

Eine ordnungsgemäße Positionierung sowie Fixierung des Kunststoffschlauchs ist für den Erfolg dieser nicht invasiven Methode unerlässlich. Es muss garantiert werden können, dass es durch die Nahrungsaufnahme und/oder die Peristaltik nicht zu einer Verschiebung des Kunststoffschlauchs kommt, so dass Abschnitte des Dünndarms, insbesondere der Zwölffingerdarms freigelegt werden und in diesem Abschnitt Nahrung resorbiert werden kann. Darüberhinaus muss sichergestellt werden, dass keine Nahrung zwischen Kunststoffschlauch und Dünndarmwand gelangen kann, was ebenfalls dazu führen würde, dass Nahrung resorbiert wird.

Aus der WO2011/062882A1 ist ein System bekannt, welches das endoskopische Positionieren des Kunststoffschlauchs im Dünndarm ermöglicht. Das System gemäß einer ersten Ausführungsvariante umfasst zwei Gehäuseteile, die aus einem biokompatiblen, im Darm/Magen auflösbaren Material, gefertigt sind, so dass sich die beiden Gehäuseteile nach einer gewissen Verweildauer im Körper des Patienten auflösen. Die beiden Gehäuseteile nehmen für den Zweck des endoskopischen Positionierens im Dünndarm/Magen den Kunststoffschlauch in einem gefalteten Zustand in sich auf, außerdem zwei ringförmige, elastische Befestigungsmittel, die zur Fixierung des Kunststoffschlauchs im Dünndarm/Magen dienen und über elastische Bänder miteinander verbunden sind.

Nach erfolgter Positionierung des einen Gehäuseteils, welcher den Kunststoffschlauch und das damit verbundene erste Befestigungsmittel umfasst, distal zum Pylorus, und des anderen Gehäuseteils, welches das zweite, mit dem ersten Befestigungsmittel oder dem Kunststoffschlauch über die elastischen Bänder verbundene zweite Befestigungsmittel umfasst, proximal des Pylorus, lösen sich die Gehäuseteile auf und geben den Kunststoffschlauch sowie die beiden Befestigungsmittel frei. Die Begriffe "distal" und "proximal" werden dabei in Bezug auf die Speiseröhre verwendet, dh. "distal" bezeichnet jene Lagen, die, bezogen auf die Speiseröhre weiter entfernt von dieser liegen und "proximal" jene Lagen, die sich näher zur Speiseröhre befinden.

Das System einer zweiten Ausführungsvariante sieht vor, dass der Kunststoffschlauch und die beiden ringförmigen, elastischen Befestigungsmittel in einem Gehäuse bzw. einer Hülle angeordnet sind, aus welcher sie nach entsprechender Positionierung im Magen-Darm-Trakt mittels einer stempelartigen Vorrichtung ausgestoßen werden.

In beiden Fällen bewirkt das mit dem Kunststoffschlauch verbundene erste Befestigungsmittel dabei eine Fixierung im Dünndarm (Zwölffingerdarm) also distal zum Pylorus und das zweite Befestigungsmittel eine Fixierung im Antrum des Magens, also proximal zum Pylorus. Zur Fixierung der Position der beiden Befestigungsmittel und damit auch des Kunststoffschlauchs im Dünndarm bzw. im Antrum des Magens üben diese einen radialen Druck auf das umgebende Gewebe aus, so dass sich diese an der Dünndarmwand bzw. an der Magenwand im Bereich dessen Antrums verspreizen. Da in diesem Bereich auch der Vagusnerv verläuft, wird dieser durch die Fixierung stimuliert, wodurch angenommen wird, dass ein Sättigungsgefühl erzeugt wird. Durch die radiale Fixierung wird sowohl eine proximale als auch eine distale Verschiebung der Befestigungsmittel erschwert. Je nach anfänglicher Positionierung können sich die Befestigungsmittel sowohl and der distalen als auch der proximalen Stirnfläche des Pylorus abstützen. Die elastischen Bänder sind dabei jedoch so ausgelegt, dass sich die beiden Befestigungsmittel sowohl vom distalen als auch vom proximalen Abschnitt des Pylorus abheben können.

Im Zuge dieses Abhebens der beiden Befestigungsmittel, insbesondere aufgrund des Abhebens des im Dünndarm (Zwölffingerdarm) angeordneten Befestigungsmittels vom distalen Abschnitt des Pylorus ist es einerseits möglich, dass Nahrung bzw. Speisebrei vom Magen seitlich an diesem Befestigungsmittel vorbei zwischen Kunststoffschlauch und Dünndarmwand gelangt und dort resorbiert wird. Andererseits wird dadurch, je nach Distanz, die sich das im Dünndarm angeordnete Befestigungsmittel vom distalen Abschnitt des Pylorus entfernt, ein Teil der Dünndarmwand freigelegt, so dass es auch entlang dieses freigelegten Abschnitts, zu einer Resorption der Nahrung kommt.

Ein weiteres Problem ergibt sich dadurch, dass die endoskopische Positionierung im Falle der ersten Ausführungsvariante des beschriebenen Systems abhängig ist von der Zeit, welche die beiden Gehäuseteile benötigen, um sich im Magen-Darm-Trakt aufzulösen. Dies birgt insbesondere Unabwägbarkeiten, welche die Sedierung oder Narkose des Patienten betreffen. Zwar kann man von einem Zeitfenster ausgehen, in welchem eine vollständige Freilegung des Kunststoffschlauchs samt Befestigungsmittel erfolgt. Je nach Zustand des Patienten kann diese jedoch auch länger oder kürzer dauern, so dass sich der intervenierende Arzt stets vergewissern muss, dass sich die Gehäuseteile auch komplett aufgelöst haben, um das gastrointestinale Implantat korrekt positionieren zu können. Ein frühzeitiges Verschieben/Entfernen des Endoskops würde dazu führen, dass sich die beiden Befestigungsmittel nicht in ihrer vorgesehen Position befinden, wenn sich die beiden Gehäuseteile vorzeitig oder verspätet auflösen.

Schlussendlich besteht bei jener Ausführungsvariante, bei welcher das gastrointestinale Implantat mittels stempelartiger Vorrichtung aus einem Gehäuse/einer Hülle ausgeschoben wird, um positioniert zu werden, das Problem, dass durch den Stempel die beiden Befestigungsmittel so zusammengedrückt werden, dass diese sich in einander verkeilen können und in Folge ihre Funktion nicht mehr erfüllen können.

### AUFGABE DER ERFINDUNG

Es ist daher eine erste Aufgabe der vorliegenden Erfindung, ein gastrointestinales Implantat vorzuschlagen, welches eine verbesserte Fixierung im Magen-/Darmtrakt ermöglicht.

Es ist eine zweite Aufgabe der Erfindung, ein gastrointestinales Implantat vorzuschlagen, das eine Resorption von Nahrung/Speisemittelbrei im Dünndarm wirkungsvoll und verlässlich verhindert.

Es ist eine dritte Aufgabe der vorliegenden Erfindung eine Vorrichtung zur transoralen/enteralen Positionierung bereitzustellen, bei welcher der Vorgang der Positionierung sicherer und exakter durchgeführt werden kann.

### DARSTELLUNG DER ERFINDUNG

Erfindungsgemäß werden die ersten beiden Aufgaben durch ein gastrointestinales Implantat gelöst, welches wie folgt umfasst:
- ein schlauchförmiges Element, ausgebildet um Speisebrei durch zumindest einen Abschnitt des menschlichen Darms zu befördern
- ein mit dem schlauchförmigen Element verbundenes erstes Befestigungsmittel mit einer ersten Anlagefläche, ausgebildet, um in einem an den Pylorus angrenzenden Abschnitt des Zwölffingerdarms positioniert zu werden,
- ein mit dem schlauchförmigen Element oder mit dem ersten Befestigungsmittel verbundenes zweites Befestigungsmittel mit einer zweiten Anlagefläche, welches dazu ausgebildet ist, im Antrum des Magens positioniert zu werden
- ein Verbindungselement, welches das erste Befestigungsmittel oder das schlauchförmige Element mit dem zweiten Befestigungsmittel verbindet,
wobei das Verbindungselement so ausgebildet ist, dass erstes und zweites Befestigungsmittel in deren Position im Zwölffingerdarm bzw. im Antrum des Magens mit deren erster und zweiter Anlagefläche unter Zwischenlage des Pylorus gegeneinander gepresst werden, ohne dass es zu einem Abheben der Befestigungsmittel vom Pylorus kommt.

Das Verbindungselement ist also hinsichtlich Länge und Elastizität so zu dimensionieren, dass es einerseits derart dehnbar ist, dass der Pylorus zunächst zwischen den beiden Befestigungsmitteln platzierbar ist. Dazu muss das Verbindungselement elastisch ausgebildet sein und die maximale mögliche Dehnlänge muss jedenfalls die Positionierung der beiden Befestigungsmittel im Dünndarm und im Antrum des Magens ermöglichen. Jedenfalls aber muss die Dimensionierung des Verbindungselementes so erfolgen, dass bei im Patienten positioniertem Implantat, von den beiden Befestigungsmitteln über deren jeweilige Anlageflächen stets Druck auf den Pylorus ausgeübt wird, so dass es zu keinem Abheben der Befestigungsmittel von der distalen bzw. proximalen Stirnfläche des Pylorus kommt, ungeachtet der stets vorhandenen Peristaltik des Magen-/Darmtraktes bzw. ungeachtet der Kräfte, die vom Speisebrei auf das gastrointestinale Implantat, insbesondere das schlauchförmige Element beim Durchtritt durch diesen, ausgeübt werden.

Gemäß einer bevorzugten Ausführungsform der Erfindung ist es vorgesehen, dass der Abstand zwischen erstem Befestigungsmittel und zweitem Befestigungsmittel im entspannten Zustand des Verbindungselementes kleiner ist als die Dicke des Pylorus, vorzugsweise kleiner als 7 mm gemessen in Richtung der vom Pylorus ausgebildeten Durchgangsöffnung.

Durch diese erfindungsgemäße Dimensionierung ist jedenfalls sichergestellt, dass die beiden Befestigungsmittel jeweils unter kontinuierlichem Druck mit deren Anlageflächen an der distalen bzw. proximalen Stirnfläche des Pylorus anliegen. Bevorzugt sind die beiden Befestigungsmittel im entspannten Zustand des Verbindungselementes weniger als 5mm voneinander entfernt, um den Pylorus durch die beiden Befestigungsmittel ausreichend fest zu klemmen, so dass ein Abheben der Befestigungsmittel von der distalen bzw. proximalen Stirnfläche des Pylorus verhindert werden kann.

Dabei kann es unter Umständen auch auf das Material des Verbindungselementes ankommen bzw. dessen Geometrie und Stärke.

Als vorteilhaft hat sich dabei Silikon als Material erwiesen. Gemäß einer weiteren bevorzugten Ausführungsform der Erfindung ist es daher vorgesehen, dass das Verbindungselement aus Silikon gefertigt ist. Silikon zeichnet sich einerseits durch seine für den vorliegenden Anwendungsfall ausreichende Elastizität aus, andererseits aber auch durch seine Verträglichkeit in Bezug auf den menschlichen Körper und einem über einen weiten Temperatur- und pH Bereich stabilen mechanischen Verhalten. Selbstverständlich sind aber auch andere biokompatible Materialien wie beispielsweise PLA, PE, Latex oder Latexersatz denkbar.

Gemäß einer weiteren bevorzugten Ausführungsform der Erfindung ist es vorgesehen, dass das Verbindungselement mehrere Bänder umfasst oder aber schlauchförmig ausgebildet ist. Während es bei der Ausbildung in Form von mehreren Bändern leichter ist, das gastrointestinale Implantat zu Falten, so bietet die Ausbildung des Verbindungselementes als Schlauch den Vorteil der einfacheren Applikation sowie Herstellung.

Gemäß einer weiteren bevorzugten Ausführungsform der Erfindung ist es vorgesehen, dass erstes und zweites Befestigungsmittel jeweils eine Durchgangsöffnung aufweisen und vorzugsweise im Wesentlichen ringförmig ausgebildet sind, wobei sich in diesem Fall auch ringförmige Anlageflächen ergeben. Dadurch können, bei entsprechender Dimensionierung des Ringdurchmessers, die auf den Pylorus wirkenden Klemmkräfte bestmöglich übertragen werden, ohne dessen Durchgangsöffnung zu blockieren oder zu verengen, wobei anzumerken ist, dass es unter Umständen gerade gewünscht sein kann, die Durchgangsöffnung des Pylorus zu verengen und damit den Nahrungsmittelfluss durch den Pylorus künstlich zu beschränken. Unter einem "ringförmigen" Befestigungsmittel wird in diesem Zusammenhang auch ein Befestigungsmittel verstanden, dessen Form von der idealen Ringform abweicht aber dennoch die Klemmung des Pylorus zwischen den Anlageflächen ermöglicht, indem diese an der proximalen bzw. distalen Stirnfläche des Pylorus anliegen.

Gemäß einer weiteren bevorzugten Ausführungsform der Erfindung ist es vorgesehen, dass das erste Befestigungsmittel durch einen ersten Endbereich des schlauchförmigen Elementes ausgebildet ist. Beispielsweise kann das erste Befestigungsmittel dadurch gebildet werden, dass das schlauchförmige Element an seinem einem Ende mehrfach aufgerollt ist, so dass sich eine Art verstärkter Kragen oder Rand ausbildet, der eine ausreichende Dicke aufweist, um eine Anlagefläche auszubilden und gegen die distale Stirnfläche des Pylorus gepresst zu werden, um diesen, gemeinsam mit dem zweiten Befestigungsmittel und dessen Anlagefläche zu klemmen. Genausogut kann der erste Endbereich des schlauchförmigen Elementes aber auch mit einer dickeren Wandstärke ausgeführt sein, als dessen restliche Länge, so dass der erste Endbereich eine Art Wulst ausbildet, die dafür vorgesehen ist, gegen die distale Stirnfläche des Pylorus gepresst zu werden.

Gemäß einer weiteren alternativen Ausführungsform der Erfindung ist es vorgesehen, dass das schlauchförmige Element einen zweiten Endbereich aufweist, der aus resorbierbarem Material gefertigt ist. Dadurch kann gewährleistet werden, dass der Schlauch zunächst, während des Positionierens im Magen-/Darmtrakt und für die Zwecke der Entfaltung an seinem zweiten Endbereich, welcher der distalen Stirnfläche des Pylorus gegenüberliegt, verschlossen ist und erst nach erfolgter Positionierung und Entfaltung für den Transport von Nahrungsbrei in hintere Dünndarmregionen durchgängig wird. Beispielsweise können der resorbierbare Abschnitt des schlauchförmigen Elementes und der nicht resorbierbare Abschnitt miteinander verklebt sein, beispielsweise durch einen Fibrinkleber.

Gemäß einer weiteren bevorzugten Ausführungsform der Erfindung ist es vorgesehen, dass die im zweiten Endbereich des schlauchförmigen Elementes vorgesehene Öffnung durch einen, vorzugsweise mittels eines in den Schlauch einleitbaren Fluids, entfernbaren Stopfens verschlossen ist. Im Zuge der Entfaltung des schlauchförmigen Elementes kann es vorgesehen sein, dieses durch Einleiten eines Fluids (beispielsweise CO2 oder Luft) über dessen ersten Endbereich im Dünndarm zu entfalten. Durch den sich im schlauchförmigen Element dadurch aufbauenden Druck wird bei dieser Ausführungsform der Stopfen nach vollkommener Entfaltung durch den Innendruck gelöst und kann auf natürliche Art und Weise ausgeschieden werden. Der Stopfen kann dabei aus beliebigem, biokompatiblem Material gefertigt sein, beispielsweise aus einem biokompatiblen Klebstoff wie Stärke oder Hartkapselgelatine. Wesentlich ist lediglich, dass dieser zunächst den für die Entfaltung erforderlichen Aufbau des Innendrucks im Schlauch ermöglicht, und danach, bei darüberhinaus gesteigertem Innendruck sich aus der Öffnung löst, so dass er auf natürlichem Weg ausgeschieden werden kann.

Gemäß einer weiteren Ausführungsform der Erfindung ist es vorgesehen, dass das schlauchförmige Element eine Strukturschwächung aufweist, beispielsweise in Form einer Perforation oder eines verdünnten Wandabschnittes, die einen zweiten Endbereich vom restlichen schlauchförmigen Element trennt. Die Strukturschwächung ist so dimensioniert, dass diese, ähnlich wie im Falle des Stopfens, im Zuge der Entfaltung des schlauchförmigen Elementes, bei genügend großem Innendruck, der beispielsweise durch ein in das schlauchförmige Element einleitbares Fluids sukzessive erhöht werden kann, nachgibt, wodurch sich der zweite Endbereich vom restlichen schlauförmigen Element abtrennen und auf natürliche Art und Weise ausgeschieden werden kann.

In einer weiteren Ausführungsform der Erfindung ist es vorgesehen, dass das das schlauchförmige Element einen zweiten Endbereich aufweist, der offen ausgebildet ist. Wie später noch erläutert werden wird, ist dies einer Entfaltung desselben nicht hinderlich.

Gemäß einer weiteren bevorzugten Ausführungsform der Erfindung ist es vorgesehen, dass erstes und zweites Befestigungsmittel, vorzugsweise deren Anlageflächen jeweils magnetisch wirkende Abschnitte umfassen, um das Gegeneinanderpressen der Befestigungsmittel unter Zwischenlage des Pylorus zu unterstützen. Die Magnetisierung der jeweiligen Abschnitte kann dabei auf unterschiedliche Art und Weise bewirkt werden. Eine bevorzugte Ausführungsform sieht vor, dass kleine Magneten im Zuge der Herstellung der Befestigungsmittel in diese eingearbeitet werden. Insbesondere im Falle der Herstellung der Befestigungsmittel aus Silikon, können im Zuge der spritzgusstechnischen Anfertigung der Befestigungsmittel die Magnete auf einfache Art und Weise in die Befestigungsmittel über den Umfang oder Abschnitte des Umfangs verteilt eingearbeitet werden. Neben dem Gegeneinanderpressen unter Zwischenlage des Pylorus wird auf diese Art und Weise auch die Stabilität der Befestigungsmittel verstärkt, da sich die Magneten an einem Befestigungsmittel gegenseitig abstoßen und so auf Distanz halten.

Erfindungsgemäß wird die dritte beschriebene Aufgabe durch
- eine Vorrichtung zur Positionierung eines gastrointestinalen Implantats mit zwei im Wesentlichen ringförmigen Befestigungsmittel, sowie
- ein System umfassend ein gastrointestinales Implantat mit zwei im Wesentlichen ringförmigen Befestigungsmittel und einer Vorrichtung zur Positionierung des gastrointestinalen Implantats
gelöst.

Dabei ist festzuhalten, dass sowohl die Vorrichtung zur Positionierung eines gastrointestinalen Implantats als auch das System nicht notwendigerweise gemeinsam mit dem zuvor beschriebenen gastrointestinalen Implantat eingesetzt werden müssen, sondern es auch vorstellbar ist, die vorgeschlagene Vorrichtung und das vorgeschlagene System mit anderen gattungsgleichen gastrointestinalen Implantaten mit im Wesentlichen ringförmigen Befestigungsmitteln einzusetzen, beispielsweise jene wie in der WO2011/062882A1 beschrieben. Die Kombination des erfindungsgemäß vorgeschlagenen gastrointestinalen Implantats bestehend aus dem Kunststoffschlauch sowie den beiden im Wesentlichen ringförmigen Befestigungsmitteln mit der ebenfalls vorgeschlagenen Vorrichtung zur Positionierung und dem vorgeschlagenen System bilden jedoch eine bevorzugte Ausführungsform/Kombination der Erfindung. Erfindungsgemäß umfasst die Positioniervorrichtung zur Positionierung eines gastrointestinalen Implantats mit zwei im Wesentlichen ringförmigen Befestigungsmitteln wie folgt:
- ein eine erste Längsachse aufweisendes äußeres Behältnis
- einen im äußeren Behältnis angeordneten, eine zweite Längsachse aufweisenden inneren Stützkörper, der das Auffädeln der ringförmigen Befestigungsmittel ermöglicht
- eine erste, zwischen äußerem Behältnis und innerem Stützkörper angeordnete Lagerstelle für das erste Befestigungsmittel des gastrointestinalen Implantats und
- eine zweite zwischen äußerem Behältnis und innerem Stützkörper angeordnete Lagerstelle für das zweite Befestigungsmittel des gastrointestinalen Implantats, wobei
- das äußere Behältnis vom inneren Stützkörper entfernbar, vorzugsweise in Richtung der zweiten Längsachse axial abziehbar ist.

Die Positioniervorrichtung ermöglicht es zunächst, die beiden Befestigungsmittel am inneren Stützkörper aufzufädeln und in einen Abstand zueinander zu bringen, der größer ist, als jener Abstand, den die beiden Befestigungsmittel im Magen-/Darmtrakt einnehmen müssen, um den Pylorus kontinuierlich zwischen sich zu klemmen. Damit die beiden Befestigungsmittel während des transoralen Einführens der Positioniervorrichtung über die Speisröhre ihre Lage zueinander und am inneren Stützkörper nicht verändern, ist das äußere Behältnis vorgesehen, das den inneren Stützkörper sowie die aufgefädelten Befestigungsmittel aufnimmt. Zwischen äußerem Behältnis und innerem Stützkörper sind Lagerstellen für die beiden Befestigungsmittel vorgesehen, welche die Lagefixierung der beiden Befestigungsmittel entsprechend dem oben erwähnten Abstand ermöglichen. Durch das äußere Behältnis, dessen Außendimensionen so gewählt sind, dass das transorale Einführen der Positioniervorrichtung durch die Speiseröhre möglich ist, erfolgt eine Komprimierung bzw. Formveränderung der Befestigungsmittel, so dass das Einbringen dieser in den Körper möglich ist.

Durch Abstimmung der Außen- und Innenabmessungen des äußeren Behältnisses und inneren Stützkörpers auf die Dicke der Befestigungsmittel kann sichergestellt werden, dass die Komprimierung/Formveränderung nicht so weit geht, dass der außerhalb des Körpers vorgegebene und eingestellte Abstand zwischen den Befestigungsmitteln sich derart verändert, dass eine Positionierung des einen Befestigungsmittels im Dünndarm und des anderen Befestigungsmittels im Antrum des Magens nicht mehr möglich ist.

Durch die Möglichkeit des Entfernens des äußeren Behältnisses vom inneren Stützkörper können sich die Befestigungsmittel aus deren Komprimierung lösen. Die Befestigungsmittel können sich dadurch entspannen, wodurch sie im Durchmesser wachsen und ihre Grundform einnehmen. Dadurch wird eine lagemäßige Ausrichtung der Befestigungsmittel distal und proximal des Pylorus ermöglicht, woraufhin der innere Stützkörper ebenfalls entfernt werden kann.

Für die Entfernung des äußeren Behältnisses existieren unterschiedliche Möglichkeiten. So kann beispielsweise das gesamte äußere Behältnis aus einem biokompatiblem, resorbierbaren (auflösbaren) Material bestehen, so dass sich das äußere Behältnis nach einer bestimmten Verweildauer im Magen-/Darmtrakt von selbst auflöst. Es ist dabei jedenfalls sicherzustellen, dass die Entfernung des äußeren Behältnisses jedenfalls erst dann erfolgt, wenn die Positioniervorrichtung den Pylorus durchragt und sich die Lagerstelle des einen Befestigungsmittels im Dünndarm (Zwölffingerdarm) und des anderen Befestigungsmittels im Antrum des Magens befindet. Nachdem die Befestigungsmittel ihre Grundform eingenommen haben, können sich diese bereits mit ihren Anlageflächen an der proximalen bzw. distalen Stirnfläche des Pylorus abstützen, so dass in weiterer Folge auch der innere Stützkörper entfernt werden kann.

Die Lagerstellen selbst können unterschiedlich ausgestaltet sein. Die Ausgestaltung der Lagerstellen hat Einfluss auf die Art und Weise der Lagefixierung der Befestigungsmittel.

In einer bevorzugten Ausführungsform ergeben sich die Lagerstellen durch simple Klemmung der Befestigungsmittel zwischen äußerem Behältnis und innerem Stützkörper. Dabei ist es vorgesehen, dass erste und zweite Lagerstelle jeweils eine äußere Begrenzung aufweisen, die durch das äußere Behältnis gebildet ist und eine innere Begrenzung aufweisen, die durch den inneren Stützkörper gebildet ist. Der Abstand zwischen den Begrenzungen ist dabei so gewählt, dass die beiden Befestigungsmittel zwischen äußerem Behältnis und innerem Stützkörper lagefixiert, dh. geklemmt sind. Anders ausgedrückt ermöglicht es die erfindungsgemäße Positioniervorrichtung durch entsprechende Dimensionierung des äußeren Behältnisses und des inneren Stützkörpers, die Befestigungsmittel zwischen sich zu klemmen, wodurch diese, wie beschrieben, komprimiert bzw. formverändert werden und transoral in den Magen-/Darmtrakt eingeführt werden können.

Um eine Optimierung der Lagerstellen im Hinblick auf die Lagefixierung zu ermöglichen, ist es gemäß einer bevorzugten Ausführungsform der Erfindung vorgesehen, dass der innere Stützkörper zwei entlang seiner Oberfläche, verlaufende Nuten zur zumindest teilweisen Aufnahme des ersten bzw. zweiten Befestigungsmittels aufweist, wobei der Mindestabstand der Nuten zueinander vorzugsweise zwischen 3 mm und 70 mm, besonders bevorzugt zwischen 5 mm und 30 mm beträgt.

Der Verlauf der Nuten entlang der Oberfläche des inneren Behältnisses kann dabei beliebig sein und ist abzustimmen auf die Befestigungsmittel, insbesondere deren Dimensionierung sowie deren erforderlichen Komprimierung und Lage. So kann es gemäß einer bevorzugten Ausführungsform vorgesehen sein, dass die Nuten in Ebenen verlaufen, die in einer Drauf- und/oder Seitenansicht rechtwinkelig auf die Längsachse des inneren Stützkörpers steht. Gemäß einer anderen Ausführungsform der Erfindung kann es aber auch vorgesehen sein, dass die Nuten in einer Drauf- und/oder Seitenansicht in Ebenen verlaufen, die schräg auf die Längsachse des inneren Stützkörpers steht. Es ist auch denkbar, dass eine der Nuten in einer Ebene verläuft, die in einer Drauf- und/oder Seitenansicht rechtwinkelig auf die Längsachse des inneren Stützkörpers steht, während die andere Nut in einer Ebene verläuft, die in einer Drauf-und/oder Seitenansicht schräg auf die Längsachse des inneren Stützkörpers steht.

Vorzugsweise ist das äußere Behältnis dabei hülsenförmig ausgebildet und - für den Fall, dass das äußere Behältnis nicht gänzlich resorbierbar ausgebildet ist, wie dies weiter oben beschrieben ist - kann es alternativ vorgesehen sein, dass der distale Endbereich des äußeren Behältnisses geschlossen und entfernbar oder aber geschlossen und öffenbar ausgebildet ist.

Während die Entfernbarkeit des distalen Endbereichs sehr einfach dadurch realisiert werden kann, dass dieser aus einem biokompatiblen, resorbierbarem (auflösefähigem) Material gefertigt ist, sieht es eine andere alternative Ausführungsform der Erfindung vor, dass das äußere Behältnis eine Strukturschwächung, vorzugsweise in Form einer Sollbruchstelle aufweist, welche die Entfernung des distalen Endbereichs vom restlichen äußeren Behältnis und damit die Öffenbarkeit durch Druckausübung auf den Endbereich, ermöglicht. Eine solche Strukturschwächung kann beispielsweise dadurch erreicht werden, dass der distale Endbereich über einen perforierten Abschnitt oder einen Abschnitt mit dünnerem Wandquerschnitt mit dem Rest des äußeren Behältnisses verbunden ist. Dadurch ist sichergestellt, dass das axiale Abziehen des äußeren Behältnisses vom inneren Stützkörper in eine Richtung erfolgen kann, die der Einführrichtung der Positioniervorrichtung entgegengesetzt ist. Das abgetrennte distale Ende kann auf natürlichem Weg aus dem Körper ausgeschieden werden.

In einer bevorzugten Ausführungsvariante der Erfindung ist es vorgesehen, dass die Strukturschwächung nicht über den gesamten Umfang des äußeren Behältnisses verläuft sondern lediglich über einen Abschnitt des Umfangs und eine Teilablösung des distalen Endbereichs ermöglicht. Der derart teilabgelöste distale Endbereich bleibt dadurch mit dem äußeren Behältnis verbunden und kann entgegen der Einführrichtung gemeinsam mit dem äußeren Behältnis wieder aus dem Körper entfernt werden.

In einer weiteren Ausführungsform der Erfindung kann es auch vorgesehen sein, dass der distale Endbereich des äußeren Behältnisses mit dem restlichen äußeren Behältnis verklebt ist und die Strukturschwächung über die Einstellung der Festigkeit des Klebstoffes erfolgt.

Damit das gastrointestinale Implantat die vorgesehene Wirkung entfalten kann, muss gewährleistet sein, dass sich das schlauchförmige Element mit seiner gesamten Länge im Darm, vorzugsweise vor der Fixierung des gastrointestinalen Implantats durch die beiden Befestigungsmitteln, entfalten kann und keine Passagen aufweist, die das Passieren des Speisebreis blockieren. Eine vollständige Entfaltung wird umso schwieriger, je länger das schlauchförmige Element ist. Ausführliche Versuche haben ergeben, dass das Vorsehen eines Ausrichtabschnitts, der ein vorzugsweise becherförmiges Aufnahmevolumen aufweist, die erforderliche, komplette Entfaltung unterstützt bzw. ermöglicht. Der Ausrichtabschnitt kann dabei das distale Ende des inneren Stützkörpers bilden, so dass das gastrointestinale Implantat mit seinem schlauchförmigen Element und seinen beiden Befestigungsmitteln vor dem transoralen Einführen der Positioniervorrichtung beginnend bei diesem Endbereich auf den inneren Stützkörper aufgefädelt werden kann, bis die beiden Befestigungsmittel ihre vorgesehenen Lagerstellen erreicht haben. Das schlauchförmige Element umhüllt zu diesem Zeitpunkt den Ausrichtabschnitt. Da der Ausrichtabschnitt eine wesentlich geringere Länge aufweist, als die Gesamtlänge des schlauchförmigen Elementes würde dieses mit einer beträchtlichen Restlänge über den Ausrichtabschnitt hinausragen. Das vorzugsweise becherförmige Aufnahmevolumen ermöglicht es jedoch, diese Restlänge im Aufnahmevolumen unterzubringen.

Um die Entfaltung des Kunststoffschlauchs zu ermöglichen, kann es vorgesehen sein, dass der innere Stützkörper einen diesen durchlaufenden Kanal aufweist, der mit einer Auslassöffnung im Ausrichtabschnitt vorgesehen ist. Über diesen Kanal kann das schlauchförmige Element mit einem Füllmedium, vorzugsweise CO₂ oder Luft beaufschlagt werden, wodurch sich dieser aus dem Aufnahmevolumen heraus in den Dünndarm entfaltet. Die Beaufschlagung mit dem Füllmedium kann dabei etwa pulsierend erfolgen oder aber mittels konstantem Druck. Bevorzugt erfolgt die Beaufschlagung mit eine Druck von 0,1 bis 9 bar, besonders bevorzugt mit nicht mehr als 4 bar.

Gemäß der Erfindung ist es vorgesehen, dass das erfindungsgemäße gastrointestinale Implantat sowie die erfindungsgemäße Vorrichtung zur Positionierung eines gastrointestinalen Implantats, ein System ausbilden, dh. gemeinsam zum Einsatz kommen. Ein solches System weist auf:
eine Vorrichtung, die wie folgt umfasst:
   - ein eine erste Längsachse aufweisendes äußeres Behältnis
   - einen im äußeren Behältnis angeordnetes, eine zweite Längsachse aufweisendes inneren Stützkörper
   - eine erste, zwischen äußerem Behältnis und innerem Stützkörper angeordnete Lagerstelle für das erste Befestigungsmittel
   - eine zweite, zwischen äußerem Behältnis und innerem Stützkörper angeordnete Lagerstelle für das zweite Befestigungsmittel, wobei
   - das äußere Behältnis vom inneren Stützkörper entfernbar, vorzugsweise in Richtung der zweiten Längsachse axial abziehbar ist
und ein gastrointestinales Implantat, das wie folgt umfasst:
   - ein schlauchförmiges Element, ausgebildet um Speisebrei durch zumindest einen Abschnitt des menschlichen Darms zu befördern
   - ein mit dem schlauchförmigen Element verbundenes, eine Durchgangsöffnung aufweisendes erstes Befestigungsmittel, ausgebildet, um in einem an den Pylorus angrenzenden Abschnitt des Zwölffingerdarms positioniert zu werden,
   - ein mit dem schlauchförmigen Element oder mit dem ersten Befestigungsmittel verbundenes, eine Durchgangsöffnung aufweisendes zweites Befestigungsmittel, welches dazu ausgebildet ist, im Antrum des Magens positioniert zu werden
   - ein Verbindungselement, welches das erste Befestigungsmittel oder das schlauchförmige Element mit dem zweiten Befestigungsmittel verbindet,
und wobei erstes und zweites Befestigungsmittel auf dem inneren Stützkörper aufgefädelt sind und in der ersten bzw. zweiten Lagerstelle lagefixiert auf eine erste Größe komprimiert sind und/oder eine erste Position einnehmen und bei Entfernung des äußeren Behältnisses auf eine zweite Größe expandieren und/oder eine von der ersten Position unterschiedliche Position einnehmen.

Das erfindungsgemäße System ermöglicht dem intervenierenden Arzt zunächst die exakte Positionierung des gastrointestinalen Implantats im Magen-/Darmtrakt des Patienten mit Hilfe der Positioniervorrichtung so, dass sich die eine Lagerstelle im Dünndarm, bzw. genauer im Zwölffingerdarm befindet und die andere Lagerstelle im Antrum des Magens. Dafür kann es vorgesehen sein, dass am äußeren Behältnis Markierungen angebracht sind, welche dem operierenden Arzt anzeigen, wie tief die erste und zweite Lagerstelle bereits in den Körper des Patienten eingeführt sind, woraus wiederum geschlossen werden kann, wo sich die Lagerstellen für die Befestigungsmittel bezogen auf den Pylorus befinden.

Kommt der intervenierende Arzt zum Schluss, dass erste und zweite Lagerstelle korrekt positioniert sind, kann der Arzt, nachdem der Schlauch entfaltet wurde, das äußere Behältnis entfernen, entweder durch axiales Abziehen des äußeren Behältnisses oder aber durch Abwarten eine Zeitspanne bis das äußere Behältnis resorbiert wurde. Nach dem das äußere Behältnis entfernt wurde, können die Befestigungsmittel expandieren bzw. eine Fixierposition einnehmen, womit die Fixierung des gastrointestinalen Implantats im Magen-/Darmtrakt abgeschlossen ist und die Vorrichtung zur Positionierung wieder entfernt werden kann.

Bevorzugte Ausführungsformen des Systems sind in den abhängigen Ansprüchen beschrieben.

### KURZE BESCHREIBUNG DER FIGUREN

Die gegenständliche Erfindung wird nun anhand eines oder mehrerer Figuren, welche Ausführungsbeispiele darstellen, näher beschrieben. Dabei zeigt:
- Fig.1: ein gastrointestinales Implantat umfassend ein schlauchförmiges Element sowie zwei Befestigungsmittel
- Fig. 2: ein gastrointestinales Implantat angeordnet im menschlichen Magen-Darm-Trakt
- Fig.3: eine Ausführungsform eines Befestigungsmittels eines gastrointestinalen Implantats mit magnetischen Abschnitten
- Fig.4: eine Ausführungsform eines Befestigungsmittels mit in seiner Länge veränderbarem Stützelement
- Fig. 5: eine Ausführungsform eines Befestigungsmittels mit Spiralfederelementen als Stützelementen
- Fig.6: eine Ausführungsform eines Befestigungsmittels mit einem Spiralfederelement als Stützelementen
- Fig. 7: eine Ausführungsform eines Befestigungsmittels
- Fig.8: eine Ausführungsform eines Befestigungsmittels
- Fig.9: eine erste Ausführungsform einer Vorrichtung zur Positionierung eines gastrointestinalen Implantats im menschlichen Magen-Darm-Trakt vor dem Entfernen des äußeren Behältnisses
- Fig.10: eine zweite Ausführungsform einer Vorrichtung zur Positionierung eines gastrointestinales Implantats im menschlichen Magen-Darm-Trakt vor dem Entfernen des äußeren Behältnisses
- Fig.11: verschiedene Ausgestaltungsmöglichkeiten von Nuten am inneren Stützkörper
- Fig.12: die erste Ausführungsform der Vorrichtung zur Positionierung eines gastrointestinalen Implantats im menschlichen Magen-Darm-Trakt mit appliziertem gastrointestinalem Implantat
- Fig.13: die zweite Ausführungsform der Vorrichtung zur Positionierung eines gastrointestinalen Implantats im menschlichen Magen-Darm-Trakt mit appliziertem gastrointestinalem Implantat
- Fig.14: eine Detailansicht eines Abschnitts des inneren Stützkörpers mit schrägen Nuten
- Fig.15: eine weitere Detailansicht eines Abschnitts des inneren Stützkörpers mit geraden Nuten
- Fig.16: eine weitere Detailansicht eines Abschnittes des inneren Stützkörpers mit Ausnehmungen
- Fig.17: eine Positioniervorrichtung samt appliziertem gastrointestinalen Implantat kurz nach dem transoralen Einführen
- Fig.18: eine Positioniervorrichtung samt appliziertem gastrointestinalen Implantat mit zurückgezogenem äußeren Behältnis
- Fig.19: eine Positioniervorrichtung samt appliziertem gastrointestinalen Implantat mit abgelöstem distalen Endbereich des schlauchförmigen Elementes
- Fig.20: eine Positioniervorrichtung samt appliziertem gastrointestinalen Implantat mit expandiertem ersten Befestigungsmittel
- Fig.21: eine Positioniervorrichtung samt appliziertem gastrointestinalen Implantat mit expandiertem zweiten Befestigungsmittel
- Fig.22: eine Ausführungsvariante eines äußeren Behältnisses mit Anschlagelement
- Fig.23: eine Ausführungsform für einen inneren Stützkörper
- Fig.24: eine Ausführungsform für einen inneren Stützkörper

### WEGE ZUR AUSFÜHRUNG DER ERFINDUNG

### GASTROINTESTINALES IMPLANTAT

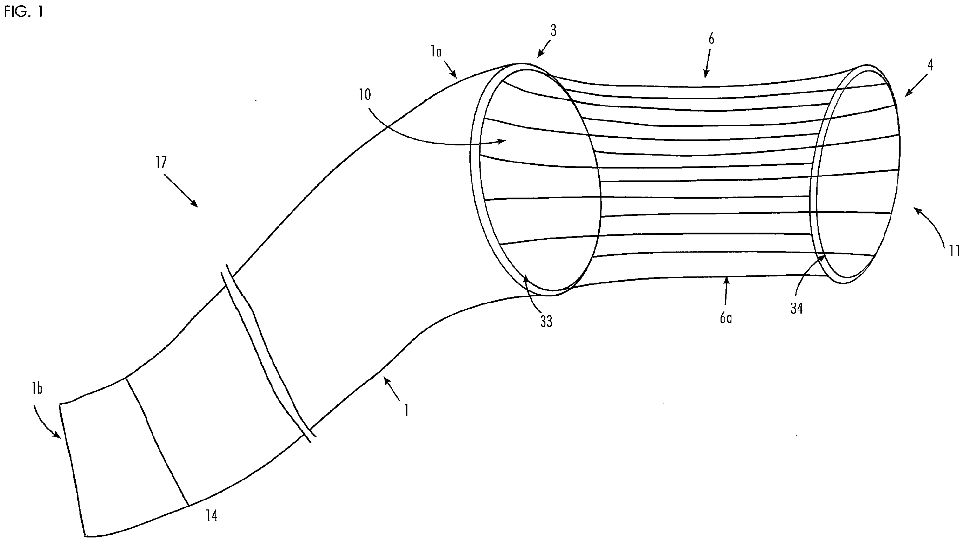

Fig.1 zeigt ein erfindungsgemäßes gastrointestinales Implantat 17 umfassend ein schlauchförmiges Element 1 sowie zwei Befestigungsmittel 3 und 4, die über ein Verbindungselement 6 miteinander verbunden sind und dazu dienen, das schlauchförmige Element 1 im Darm zu positionieren. Es hat die Aufgabe, den über den Magen in den Darm eintretenden Speisebrei durch zumindest einen Abschnitt des menschlichen Darms zu befördern, ohne dass es zur Verdauung des Speisbreis kommt. Bevorzugt ist das schlauchförmige Element 1 zumindest abschnittsweise aus Silikon oder Fluorsilikon oder einem anderen biokompatiblen Material gefertigt, welches in der Lage ist, länger im Magen oder Darm zu verweilen ohne sich zu zersetzen und ausreichend Flexibilität aufweist, um über den Mund und die Speiseröhre, vorzugsweise in gefaltetem/invaginiertem Zustand in den Magen-/Darmtrakt befördert zu werden.

Die Länge des schlauchförmigen Elementes 1 kann je nach Einsatzzweck variieren. Je länger das schlauchförmige Element 1 ausgebildet ist, desto länger ist jener Bereich des Darms, durch welchen der Speisebrei ohne Resorption befördert werden kann und desto größer und schneller zu erreichen ist die zu erzielende Gewichtsreduktion. Auch die Wanddicke des schlauchförmigen Elementes 1 kann abschnittsweise variieren, um gegebenenfalls die Widerstandsfähigkeit gegenüber den natürlichen Peristaltikbewegungen des Magen-/Darmtraktes zu erhöhen.

Fig. 1 zeigt das schlauchförmige Element 1 in einem entfalteten Zustand. Sein eines (distales) Ende 1b ist daher - zunächst - verschlossen und an seinem anderen (proximalen) Ende 1a ist das schlauchförmige Element mit dem ersten Befestigungsmittel 3 dicht verbunden bzw. einstückig gefertigt, so dass zwischen Befestigungsmittel 3 und schlauchförmigem Element 1 kein Speisebrei durchtreten kann. Wie später noch genauer beschrieben wird, dient das verschlossene Ende 1b zunächst dazu, das schlauchförmige Element 1 auf seine vorgesehene Länge im Darm zu entfalten.

Die Lagefixierung des schlauchförmigen Elementes 1 im Darm erfolgt über die Befestigungsmittel 3 und 4 bzw. deren Anlageflächen 33 (Befestigungsmittel 3) und 34 (Befestigungsmittel 4), welche dazu dienen, den Pylorus 7 zwischen sich zu klemmen und derart eine Verankerung des gastrointestinalen Implantats im Magen-/Darmtrakt zu ermöglichen. Um die Klemmfunktion auszuüben ist es vorgesehen, dass die beiden Befestigungsmittel 3 und 4 über ein Verbindungselement 6 miteinander verbunden sind. Das Verbindungselement 6 ist elastisch ausgebildet und aus gefertigt und besteht bevorzugt aus einer Anzahl an Bändern 6a oder alternativ aus einem schlauchförmigen Verbindungselement (nicht dargestellt). Sowohl Bänder 6a als auch das schlauchförmige Verbindungselement sind ausgebildet, um durch die Durchtrittsöffnung des Pylorus geführt zu werden. Die Elastizität des Verbindungselementes 6 ist dabei so gewählt, dass bei Überschreiten eines Abstandes zwischen den beiden Anlageflächen 33 und 34, welcher Abstand etwas geringer ist als die übliche Dicke des Pylorus, eine Zugkraft auf die beiden Befestigungsmittel 3,4 und damit die Anlageflächen 33 und 34 wirkt, welche - ungeachtet der durch die Peristaltik verursachten Bewegungen des Magen/Darmtraktes bzw. ungeachtet der Schließ- und Öffnungsbewegungen des Pylorus - das Lösen der jeweiligen Anlagefläche 33,34 von der distalen bzw. proximalen Stirnfläche des Pylorus verhindert, so dass die Anlageflächen 33,34 mit kontinuierlichem Druck an den Stirnflächen 31,32 des Pylorus anliegen.

Bevorzugterweise sind auch die Befestigungsmittel 3,4 ausreichend elastisch ausgebildet, so dass eine Anpassung an die unterschiedlichen Dimensionen des Magen-/Darmtrakts, sowie der bereits erwähnten Bewegungen aufgrund der Peristaltik und der Schließ- und Öffnungsbewegungen des Pylorus möglich ist. Dabei ist es vorgesehen, dass die beiden Befestigungsmittel 3,4 im entspannten Zustand annähernd ringförmige Gestalt aufweisen, so dass einerseits ausreichend Kontaktfläche vorhanden ist, um den Pylorus beidseitig zu klemmen, die Befestigungsmittel 3,4 aber andererseits auch eine ausreichend große Durchflussöffnung 10,11 aufweisen, um den ungehinderten Übertritt des Speisebreis in den Dünndarm, konkret Zwölffingerdarm zu ermöglichen. Anzumerken ist, dass aber auch größere, temporäre Abweichungen von der kreisförmigen Gestalt die Funktionalität der Befestigungsmittel 3,4 nicht wesentlich einschränken. Bevorzugt sind die Befestigungsmittel 3,4 kreisförmig ausgebildet.

In einer alternativen Ausführungsform der Erfindung kann es vorgesehen sein, dass der die Durchflussöffnung 10,11 definierende Innendurchmesser der annähernd ringförmigen Befestigungsmittel 3,4 nicht größer als 5mm ist, so dass der Übertritt des Speisebreis vom Magen in den Darmtrakt verzögert wird, um die raschere Einstellung eines Sättigungsgefühls zu ermöglichen.

Bevorzugt sind sowohl die beiden Befestigungsmittel 3,4 als auch das Verbindungselement 6 aus dem gleichen Material gefertigt wie das schlauchförmige Element 1. Aufgrund der erfindungsgemäßen Anforderungen an das Verbindungselement 6 ist es aber auch denkbar, dass dieses aus einem zu den Befestigungsmitteln 3,4 unterschiedlichen Material besteht.

Grundsätzlich sind allerdings alle Materialien zur Ausbildung der Befestigungsmittel 3,4 bzw. des Verbindungselementes 6 denkbar, die ausreichend elastisch und körperverträglich sind.

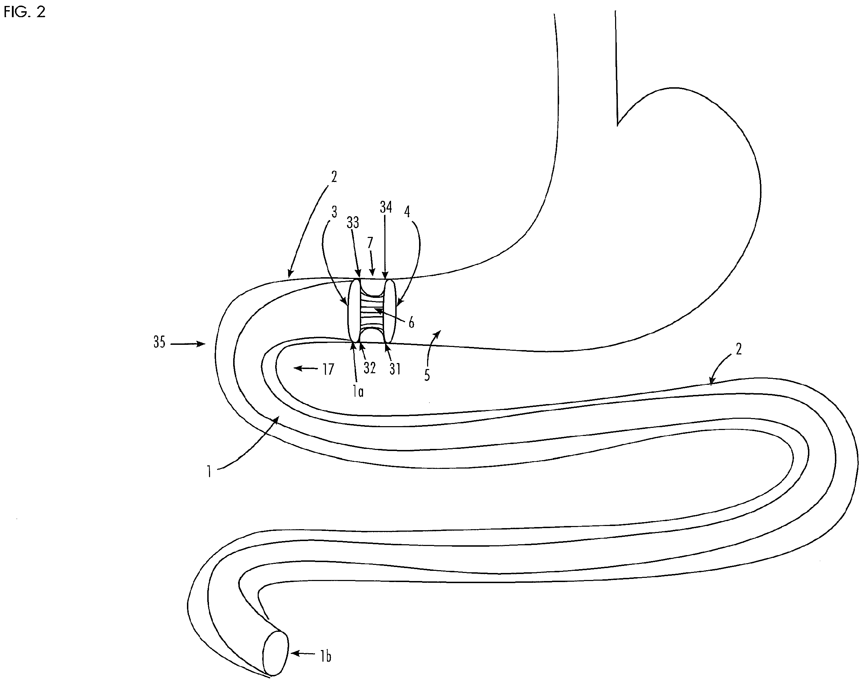

Fig.2 zeigt das gastrointestinale Implantat in seiner Endposition im Magen-/Darmtrakt angeordnet. Das schlauchförmige Element 1 befindet sich dabei bereits vollständig entfaltet im Darm 35 mit seinem proximalen Ende 1a im Zwölffingerdarm 2 beginnend. Die beiden Befestigungsmittel 3,4 sind jeweils distal bzw. proximal zum Pylorus 7 angeordnet. Das Verbindungselement 6, welches aus mehreren Bändern 6a besteht, ist so ausgebildet, dass es in der eingezeichneten Endposition des gastrointestinalen Implantats 17 die beiden Befestigungsmittel 3,4 zueinander zieht, so dass deren Anlageflächen 33,34 kontinuierlich an der proximalen 31 bzw. distalen 32 Stirnfläche des Pylorus 7 anliegen und diesen dauerhaft und beidseitig zwischen sich klemmen , ohne dass es zu einem Abheben der Anlageflächen 33,34 von der proximalen 31 und/oder 32 distalen Stirnfläche des Pylorus 7 kommt.

Das distale Ende 1b des schlauchförmigen Elementes 1 ist in dieser Endposition bereits offen, so dass Speisebrei das schlauchförmige Element 1 zur Gänze passieren kann.

Die beiden Befestigungsmittel 3,4 können magnetisch wirkende Abschnitte 29,30 aufweisen, welche derart wirken, dass sie die Zugkraft des Verbindungselementes 6 und damit die Klemmwirkung unterstützen. Darüberhinaus unterstützen die magnetischen Abschnitte 29,30 die Lagefixierung der Befestigungsmittel 3,4 derart, dass die Durchflussöffnungen 10,11 ungeachtet der stattfindenden Peristaltik und Öffen- und Schließbewegungen des Pylorus 7 stets im Wesentlichen konzentrisch ausgerichtet bleiben bzw. im Falle von verformten, nicht kreisrunden Durchflussöffnungen, so ausgerichtet bleiben, dass der Durchfluss des Speisebreis nicht behindert oder gar blockiert wird. Das, auch temporäre, radiale Verrutschen der Befestigungsmittel 3,4 kann dadurch verhindert werden.

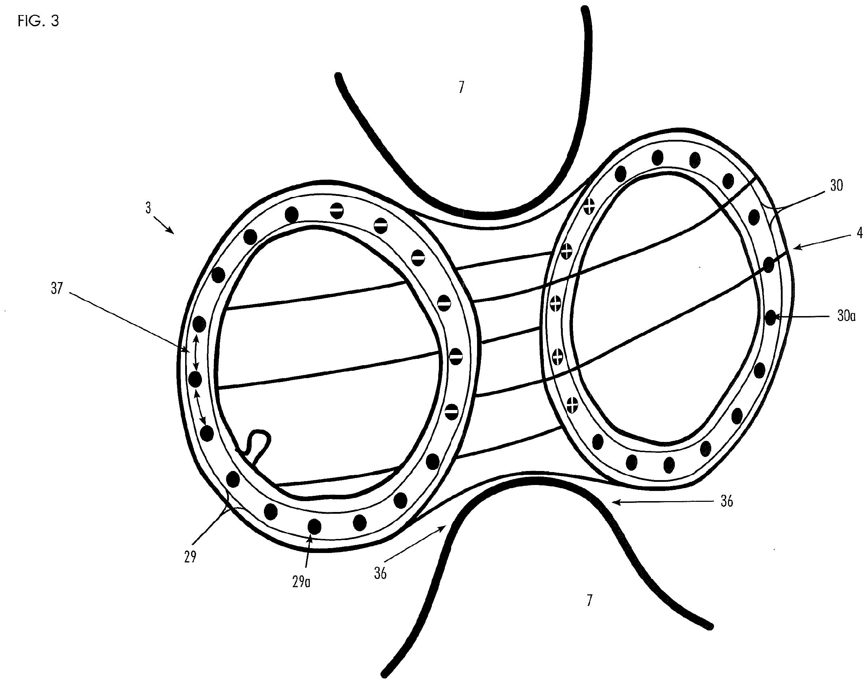

Fig.3 zeigt Befestigungsmittel 3,4 mit magnetischen Abschnitten 29,30, wobei in diesem Ausführungsbeispiel jedes der Befestigungsmittel 3,4 jeweils einen magnetischen Abschnitt 29,30 aufweist, der den gesamten Umfang des Befestigungsmittels 3,4 umspannt. Es ist aber auch denkbar, dass an jedem Befestigungsmittel 3,4 mehrere magnetische Abschnitte vorgesehen sind, die dann entsprechend kleiner dimensioniert sind und beispielsweise durch nicht magnetisch wirkende Abschnitte voneinander getrennt sind.

Im dargestellten Ausführungsbeispiel umfasst jeder Abschnitt 29,30 mehrere Einzelmagnete 29a,30a, die so angeordnet sind, dass sich die Einzelmagnete 29a des ersten Befestigungsmittels 3 und die Einzelmagnete 30a des zweiten Befestigungsmittels 4 anziehen und damit die Klemmwirkung unterstützen (siehe Pfeil mit Bezugszeichen 36) .

Gleichzeitig ermöglicht die dargestellte Anordnung, dass sich die Einzelmagnete 29a bzw. 30a gegenseitig abstoßen (siehe Pfeil mit Bezugszeichen 37). Dadurch ist es möglich die vorgegebene Form, vorzugsweise die vorgegebene Ringform zu stützen und Formveränderungen, die durch das Zusammendrücken der Befestigungsmittel 3,4 entstehen würden (beispielsweise aufgrund der Peristaltik), beispielsweise ungewünschte Torsionen, entgegenzuwirken.

Unabhängig davon kann die Oberfläche der Befestigungsmittel 3,4 auch aufgerauht sein, um den Torsionswiderstand zu erhöhen.

Das es sich beim Pylorus 7 oder Magenpförtner um einen Ringmuskel handelt, ist es, wie bereits erwähnt, von Vorteil, wenn die beiden Befestigungsmittel 3,4 in ihrer Endposition im Magen-/Darmtrakt, in welcher das gastrointestinale Implantat 17 fixiert ist, ebenfalls annähernd Ringform annehmen, so dass sich deren Anlageflächen 33,34 jeweils bestmöglich an die proximale bzw. distale Stirnfläche 31,32 des Pylorus 7 anlegen können. Dabei gilt es zu beachten, dass für die Zwecke des transoralen Einführens des gastrointestinalen Implantats 17 über die Speiseröhre, die Befestigungsmittel 3,4 zunächst zumindest abschnittsweise komprimiert werden müssen, was in der Regel auch mit einer Formveränderung einhergeht. Die grundsätzlich elastische Ausgestaltung der Befestigungsmittel 3,4 ermöglicht einerseits deren erforderliche Komprimierung und Formänderung, andererseits aber auch deren Expansion in einen Endzustand, in welchem die Klemmung des Pylorus erfolgen soll.

In einer ersten Ausführungsform ist es dabei vorgesehen, dass die Befestigungsmittel 3,4 jeweils aus einem elastischen Ring aus biokompatiblem Material wie beispielsweise Silikon, Fluorsilikon oder dergleichen bestehen, welches den oben beschriebenen Anforderungen an die Elastizität genügt.

In einer weiteren, alternativen Ausführungsform ist es vorgesehen, dass der elastische Ring eines oder beider Befestigungsmittel 3,4 aus biokompatiblem Material wie beispielsweise Silikon, Fluorsilikon oder dergleichen gefertigt ist und ein Innenvolumen aufweist, in welchem ein Stützelement 41, zB. aus Polyethylen (PE) angeordnet ist.

Das Stützelement 41 hat die Aufgabe, das Befestigungsmittel 3,4 in seiner vorgesehenen Endposition formstabiler zu gestalten und so die Klemmwirkung zu optimieren.

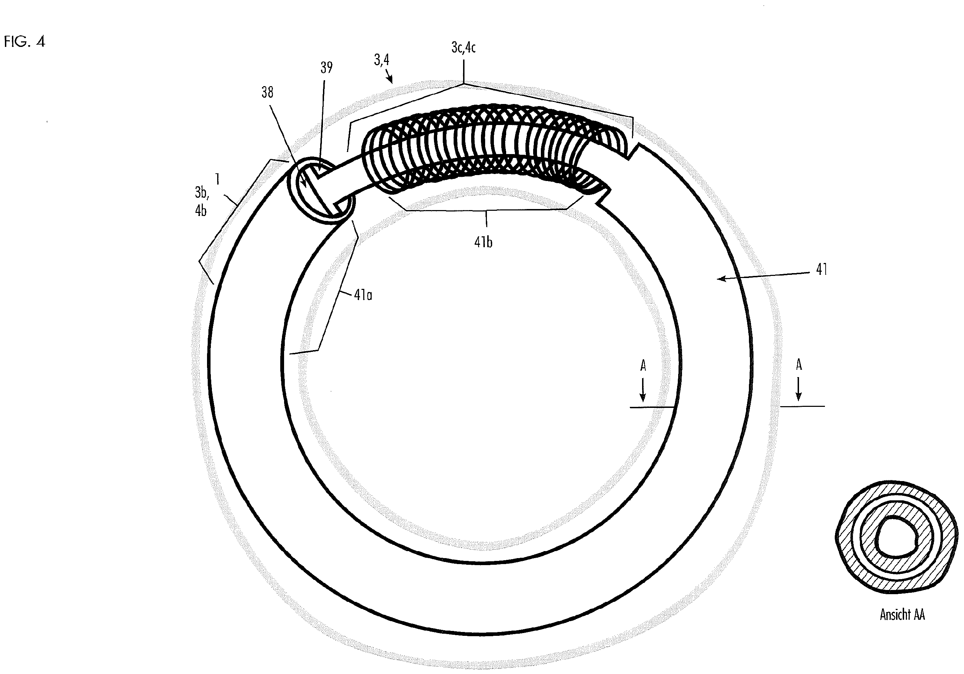

Um dennoch eine ausreichende Komprimierung während des transoralen Einführens des gastrointestinalen Implantats zu ermöglichen kann es gemäß dieser alterativen Ausführungsform vorgesehen sein, dass ein solches Stützelement 41 geteilt ausgebildet ist und einen ersten Endabschnitt 41a mit einem Innenvolumen 38 aufweist, in welchem der sich durch die Teilung ergebende zweiten Endabschnitt 41b verschiebbar gehalten ist. Der zweite Endabschnitt 41b weist zu diesem Zwecke einen gegenüber dem restlichen Stützelement 41 geringeren Querschnitt auf, so dass er problemlos in den für diese Zwecke ein Innenvolumen 38 aufweisenden ersten Endabschnitt 41a einführbar ist, wie dies in Fig.4 ersichtlich ist.

Auf diese Art und Weise sind die Befestigungsmittel 3,4 leichter komprimierbar und derart besser an die Peristaltik und Öffen- und Schließbewegung des Pylorus 7 anpassbar.

Um das Klemmvermögen eines Befestigungsmittels 3,4 dieser Ausführungsform zu optimieren, kann es vorgesehen sein, dass der Endabschnitt 41b von einem Spiralfederelement 40 umgeben ist, welches den Endabschnitt 41b stets weitest möglich aus dem Innenvolumen 38 zieht, um den Umfang des ringförmigen Befestigungsmittel 3,4 stets so groß wie möglich zu halten und damit die größtmögliche Anlagefläche 33, 34 zum Klemmen der Stirnflächen 31,32 des Pylorus 7 zur Verfügung zu stellen.

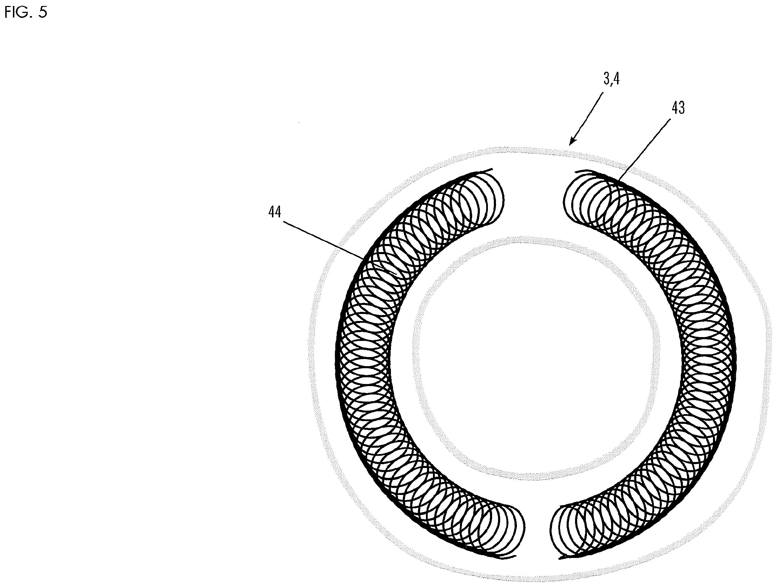

Fig.5 zeigt eine Ausführungsform eines Befestigungsmittels 3,4 ähnlich jener in Fig.4 dargestellten Ausführungsform mit dem Unterschied, dass es anstelle des geteilten Stützelementes 41 mit ineinanderschiebbaren Endabschnitten 41a und 41b zwei Spiralfederelemente 43,44 aufweist, welche als Stützelemente fungieren.

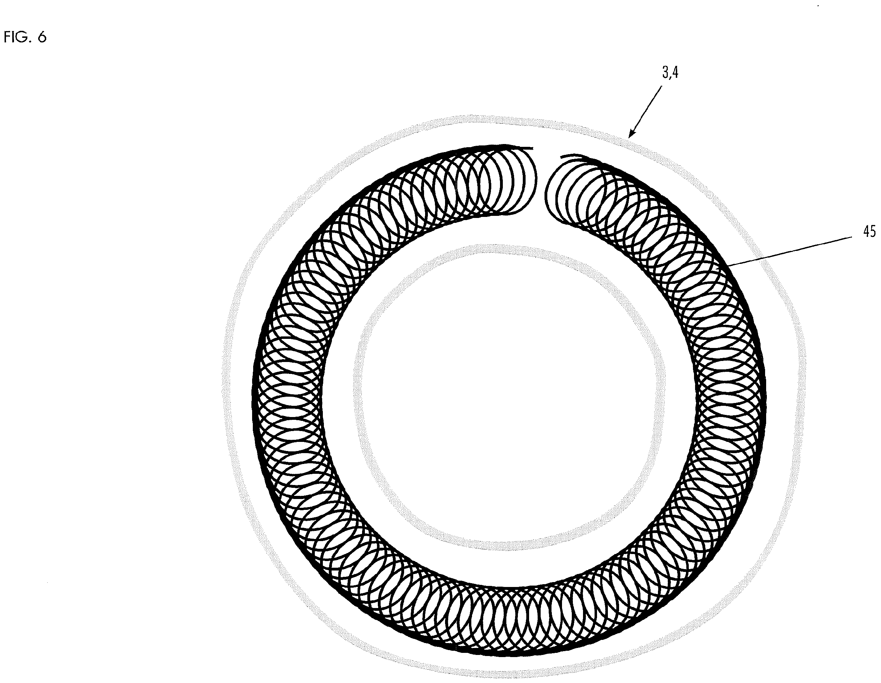

Alternativ dazu kann auch lediglich ein einziges Spiralfederelement 45 vorgesehen sein, welches entsprechend dem Stützelement 41 der Ausführungsform gemäß Fig.4, geteilt ausgeführt ist, wie dies in Fig.6 dargestellt ist.

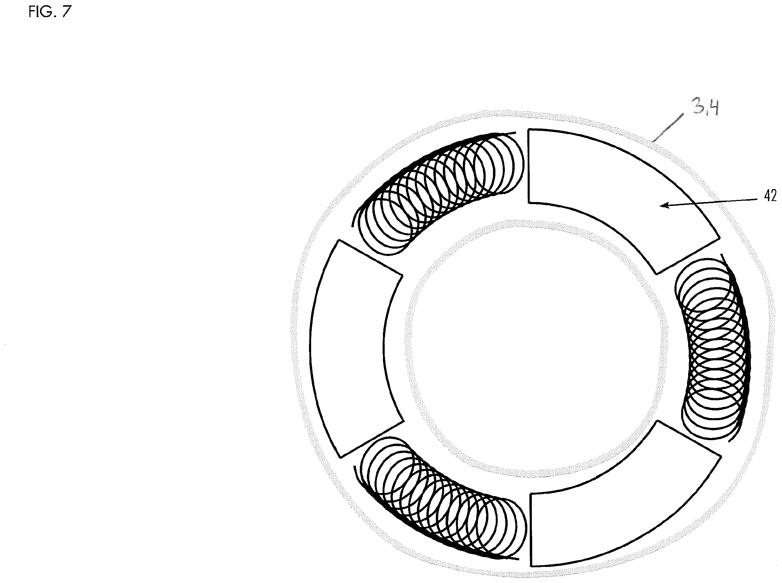

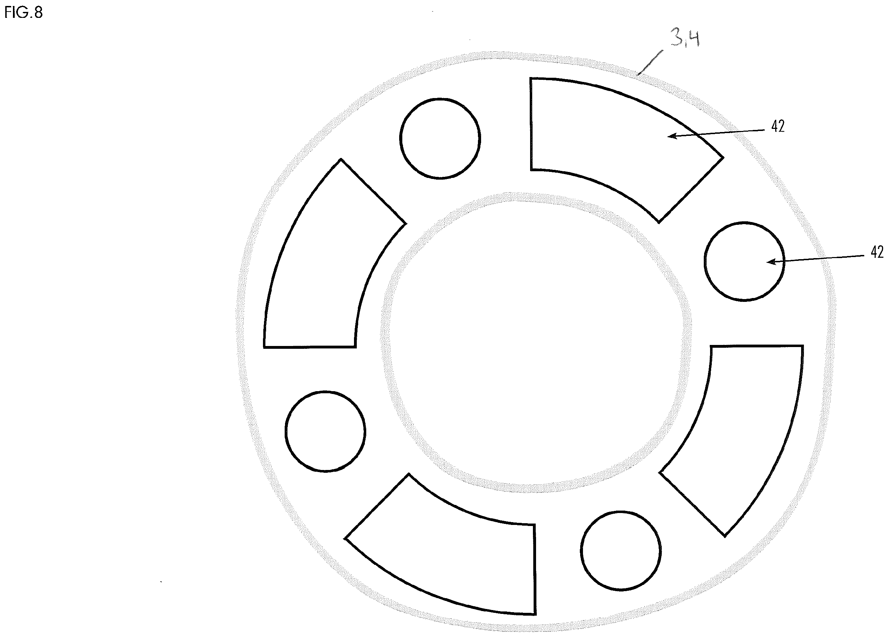

Fig.7 und Fig.8 zeigen jeweils alternative Ausführungsformen eines Befestigungsmittels 3,4 mit zusätzlichen Stützelementen 42 beispielsweise aus PE (Polyethylen).

### POSITIONIERVORRICHTUNG ZUR POSITIONIERUNG EINES GASTROINTESTINALEN IMPLANTATS IM MAGEN-/DARMTRAKT SOWIE SYSTEM UMFASSEND EIN GASTROINTESTINALES IMPLANTAT UND EINE POSITIONIERVORRICHTUNG

Um das gastrointestinale Implantat wie in den Fig.1 bis 8 beschrieben in seine Endposition im Magen-/Darmtrakt zu befördern, ist eine Positioniervorrichtung 18 vorgesehen. Die im folgenden beschriebene Positioniervorrichtung 18 kann grundsätzlich auch für die Positionierung anderer gastrointestinalen Implantate, die sich von jenen in den Fig.1 bis 8 beschriebenen unterscheiden, eingesetzt werden soferne diese anderen gastrointestinalen Implantate zwei im Wesentlichen ringförmige Befestigungsmittel aufweisen, die zur Anlage an der distalen sowie proximalen Stirnfläche des Pylorus vorgesehen sind, insbesondere auch für gastrointestinale Implantate, bei welchen das Verbindungselement 6 nicht erfindungsgemäß ausgebildet ist und daher ein Abheben der Anlageflächen 33,34 von der distalen bzw. proximalen Stirnfläche 31,32 des Pylorus 7 ermöglicht.

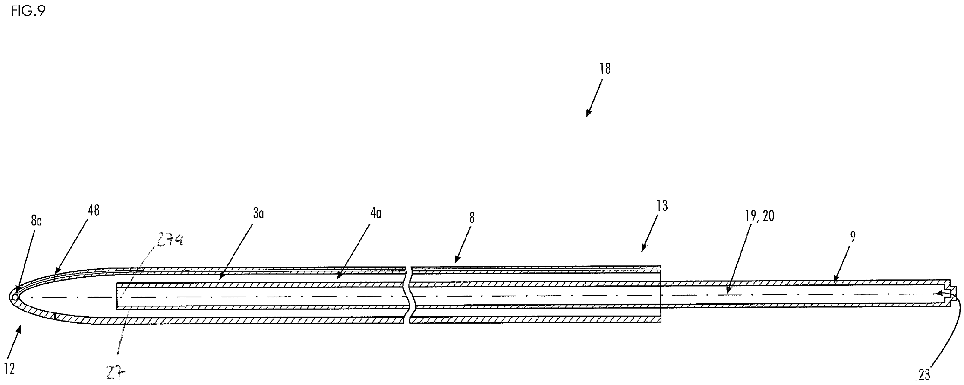

Fig.9 zeigt eine erfindungsgemäße Positioniervorrichtung bestehend aus einem äußeren Behältnis 8 mit einem Innenvolumen, in welchem ein innerer Stützkörper 9 angeordnet ist. Das äußere Behältnis 8 weist die Möglichkeit 8a zur Befestigung eines Führungsdrahtes auf, auf dessen Zweck noch später eingegangen wird.

Der distale Endbereich 12 des äußeren Behältnisses 8, also jener Endbereich, der im Zuge des transoralen Einführens in die Speiseröhre vorne liegend angeordnet ist, ist für die Zwecke des Einführens abgerundet, patronenförmig geformt und geschlossen aber entfernbar oder zumindest öffenbar ausgebildet. Die Entfernbarkeit kann dadurch erreicht werden, dass der Endbereich 12 aus einem biokompatiblen, resorbierbarem (auflösefähigem) Material gefertigt ist. Die Öffenbarkeit wird dadurch ermöglicht, dass das äußere Behältnis eine Strukturschwächung, vorzugsweise in Form einer Sollbruchstelle aufweisen kann, welche die Entfernung des distalen Endbereichs 12 vom restlichen äußeren Behältnis durch Druckausübung auf den Endbereich 12 von innen, ermöglicht. Eine solche Strukturschwächung kann beispielsweise dadurch erreicht werden, dass der distale Endbereich 12 über einen perforierten Abschnitt oder einen Abschnitt mit dünnerem Wandquerschnitt mit dem Rest des äußeren Behältnisses 8 verbunden ist. In einer bevorzugten Ausführungsvariante der Erfindung ist es vorgesehen, dass die Strukturschwächung nicht über den gesamten Umfang des äußeren Behältnisses 8 verläuft sondern lediglich über einen Abschnitt des Umfangs und eine Teilablösung des distalen Endbereichs ermöglicht.

Der proximale Endbereich 13 dient zum Einbringen des inneren Stützkörpers 9 und weist in einer bevorzugten Ausführungsform Anschlagmittel (nicht gezeichnet) auf, um äußeres Behältnis 8 und inneren Stützkörper 9 in eine definierte Lage zueinander zu bringen.

Der innere Stützkörper 9 weist vorzugsweise einen kreisförmigen Querschnitt auf, um das Auffädeln der Befestigungsmittel 3,4 zu ermöglichen.

Der Abstand zwischen äußerem Behältnis 8 und innerem Stützkörper 9 ist so gewählt, dass die Befestigungsmittel 3,4 zwischen den beiden Bauteilen geklemmt werden können und damit lagefixiert werden. Auf diese Art und Weise bilden sich eine erste Lagerstelle 3a für das erste Befestigungsmittel 3 und eine zweite Lagerstelle 4a für das zweite Befestigungsmittel 4 aus. Diese sind rein beispielsweise in Fig.9 eingezeichnet.

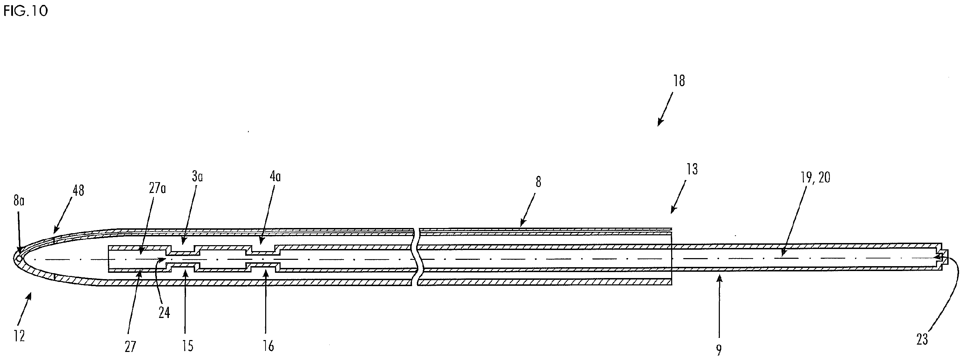

In einer alternative Ausführungsform wie in Fig.10 gezeigt, sind erste und zweite Lagerstelle 3a,4a durch Nuten 15,16 an der Oberfläche des inneren Stützkörpers 9 gebildet, in welcher die beiden Befestigungsmittel 3,4 zumindest teilweise aufnehmbar sind, dh. die Befestigungsmittel 3,4 können entweder ganz in den Nuten 15,16 angeordnet sein oder aber nur teilweise und über diese hinaussragen. Der Abstand der Nuten 15,16 zueinander beträgt vorzugsweise zwischen 3mm und 70mm, besonders bevorzugt zwischen 5mm und 30mm. Die Ausbildung der Lagerstellen 3a,4a als Nuten hat den Vorteil, dass die Lagefixierung nicht reibschlüssig erfolgt sondern mittels Formschluss, wodurch ein versehentliches Verrutschen der Befestigungsmittel 3,4 im Prinzip ausgeschlossen werden kann und auch der exakte Abstand zwischen den Befestigungsmitteln 3,4 am inneren Stützkörper 9 leichter einstellbar ist.

Der Verlauf der Nuten 15,16 an der Oberfläche des inneren Stützkörpers 9 ist abzustimmen auf die Ausführungsform und/oder Dimensionierung und/oder Elastizität der Befestigungsmittel 3,4, sowie den erforderlichen/gewünschten Grad der Komprimierung.

So kann es gemäß einer bevorzugten Ausführungsform der Erfindung vorgesehen sein, dass die Nuten 15,16 in Ebenen verlaufen, die in einer Drauf- und/oder Seitenansicht rechtwinkelig zur Längsachse 20 des inneren Stützkörpers 9 angeordnet sind. Gemäß einer anderen Ausführungsform der Erfindung kann es aber auch vorgesehen sein, dass die Nuten 15,16 in einer Drauf- und/oder Seitenansicht in Ebenen verlaufen, die schräg zur Längsachse 20 des inneren Stützkörpers 9 angeordnet sind. Es ist auch denkbar, dass eine der Nuten in einer Ebene verläuft, die in einer Drauf- und/oder Seitenansicht rechtwinkelig zur Längsachse 20 des inneren Stützkörpers 9 verläuft, während die andere Nut in einer Ebene verläuft, die in einer Drauf- und/oder Seitenansicht schräg zur Längsachse 20 des inneren Stützkörpers 9 verläuft. Die Schrägstellung der Nuten 15,16 ermöglicht einen geringeren Grad der Kompression der Befestigungsmittel 3,4.

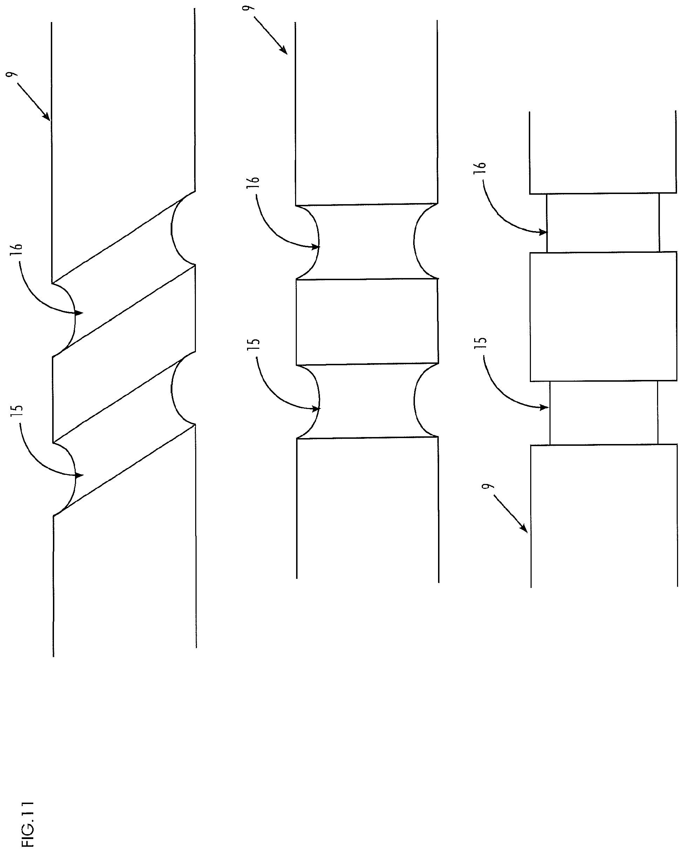

Fig.11 zeigt beispielhaft verschiedene Ausgestaltungsmöglichkeiten der Nuten 15,16 am inneren Stützkörper 9.

Die Lagerstellen 3a,4a können auch durch eine Kombination der beiden oben beschriebenen Ausführungsformen gebildet werden, so dass die abschnittsweise in den Nuten 15,16 aufgenommenen Befestigungsmittel 3,4 gleichzeitig zwischen innerem Stützkörper 9 und äußerem Behältnis 8 geklemmt werden.

Unabhängig von der konkreten Ausgestaltung der Lagerstellen ist der Abstand zwischen den Lagerstellen 3a,4a stets so gewählt, dass dieser etwas größer als die Dicke des Pylorus 7 ausgebildet ist und das Verbindungselement 6, wenn sich die beiden Befestigungsmittel 3,4 an den Lagerstellen 3a,4a befinden, bereits gespannt ist und eine Zugkraft auf jedes der beiden Befestigungsmittel 3,4 ausübt.

Mit Bezug zu Fig. 9 und 10 kann es gemäß den dort dargestellten Ausführungsformen vorgesehen sein, dass der innere Stützkörper 9 an seinem distalen Endbereich 22 einen Ausrichtabschnitt 27 für das schlauchförmige Element 1 aufweist, wie dies konkret in Fig.10 dargestellt ist. Der Ausrichtabschnitt 27 dient dazu das schlauchförmige Element 1 zunächst in einem kompakten platzsparenden Zustand aufnehmen zu können, um das transorale Einführen des gastrointestinalen Implantats zu ermöglichen bzw. zu erleichtern. In weiterer Folge dient der Ausrichtabschnitt 27 aber auch dazu, die vollständige Entfaltung des schlauchförmigen Elementes 1 aus dem kompakten Zustand zu ermöglichen.

Zu diesem Zweck kann der Ausrichtabschnitt 27 gemäß einer bevorzugten Ausführungsform der Erfindung mit einem vorzugsweise becherförmigen Aufnahmevolumen 27a versehen sein, wie dies in den Fig.9 und 10 dargestellt ist, wobei das offene Ende des Aufnahmevolumens 27a in Richtung distales Ende des äußeren Behältnisses 8 weist.

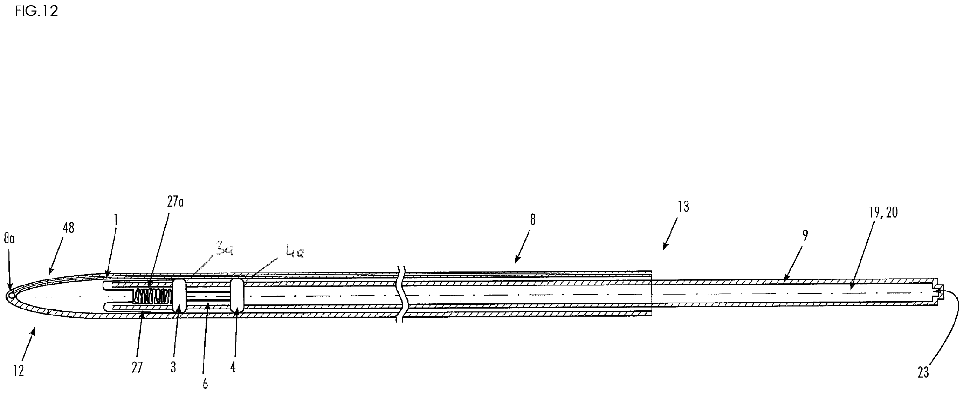

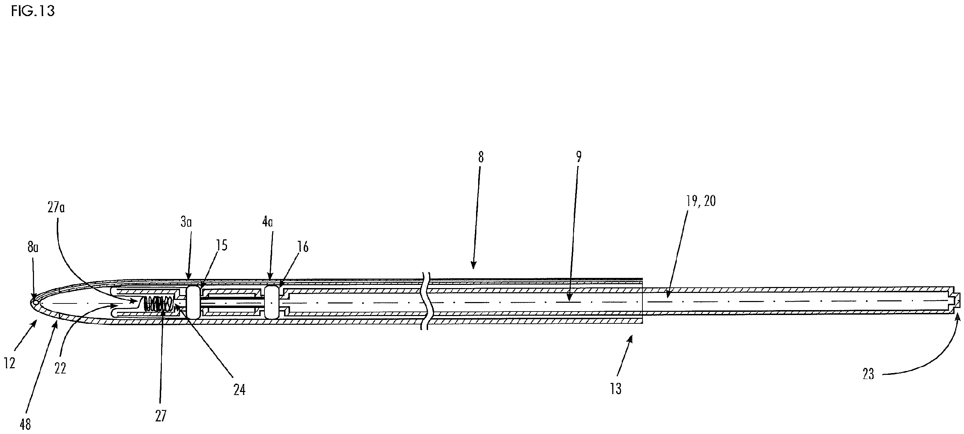

Die Applikation eines gastrointestinalen Implantats an der Positioniervorrichtung 18 ist in den Fig. 12 und 13 gezeigt, wobei Fig.12 die Applikation an einer Positioniervorrichtung 18 zeigt, wie sie in Fig.9 dargestellt ist und Fig.13 die Applikation an einer Positioniervorrichtung 18, wie sie in Fig.10 dargestellt ist.

Dabei wird das gastrointestinale Implantat 17, wie es beispielsweise in den Fig.1 bis 8 dargestellt ist, zunächst auf den inneren Stützkörper 9 aufgefädelt. Für den Fall, dass der innere Stützkörper 9 mit Nuten 15,16 versehen ist, werden die Befestigungsmittel 3,4 mit zumindest einem Teil ihres Querschnitts in diesen Nuten 15,16 gelagert, welche die Lagerstellen 3a,4a ausbilden. Unabhängig davon, ob die Lagerstellen 3a,4a durch Nuten 15,16 ausgebildet sind oder aber alleine durch den Abstand zwischen äußerem Behältnis 8 und innerem Stützkörper 9, wie dies in Fig.12 dargestellt ist, sind die Befestigungsmittel 3,4 in diesem Zustand komprimiert, da das äußere Behältnis 8 deren Expansion auf einen größeren Durchmesser verhindert.

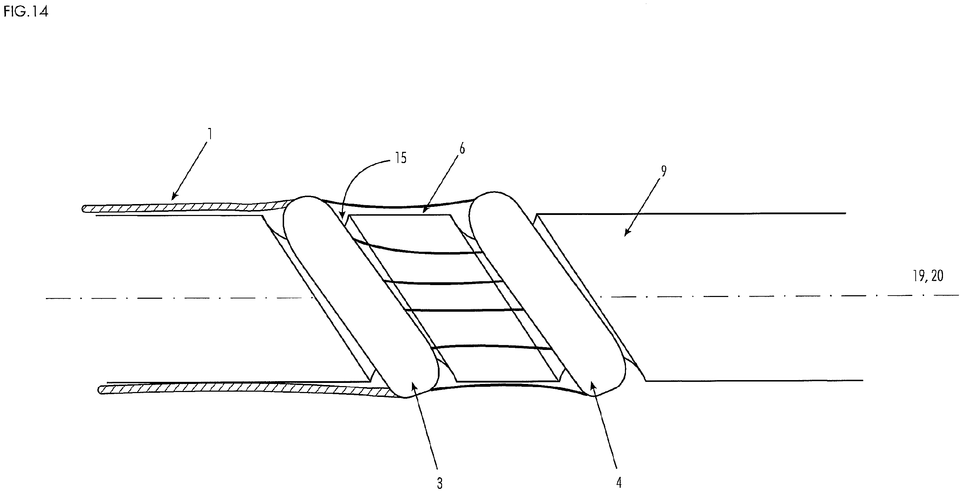

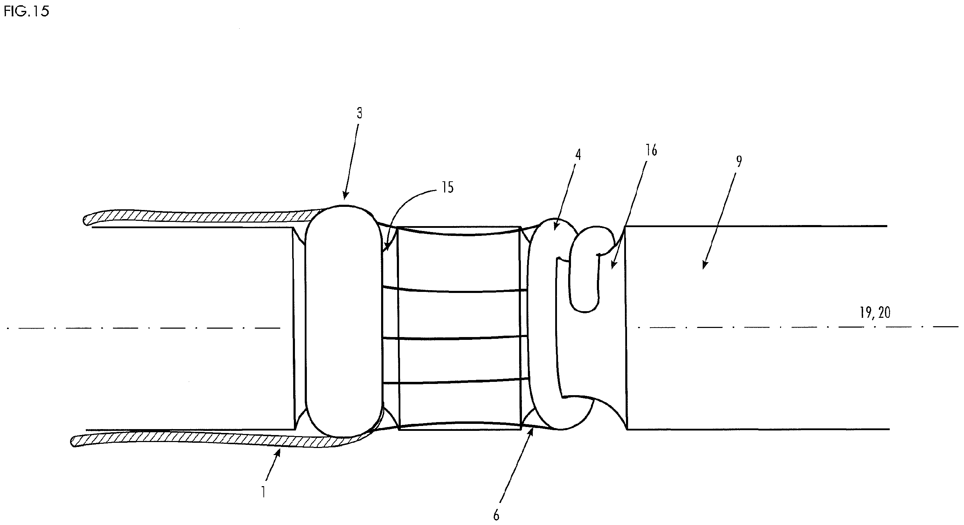

Fig.14 und 15 zeigen rein beispielhaft den Bereich der Nuten 15,16 in vergrößerter Darstellung, wobei die Nuten 15,16 in Fig.14 schräg zur Längsachse 20 ausgeführt sind und in Fig.15 gerade. Ebenfalls rein beispielhaft ist in Fig. 15 das eine Befestigungsmittel 4 spiralförmig ausgebildet, um zu demonstrieren, dass grundsätzlich die Befestigungsmittel 3,4 auch andere als die bislang dargestellten Formen aufweisen können. Während in den dargestellten Ausführungsformen Befestigungsmittel 3,4 kreisförmigen Querschnitt aufweisen, sind auch andere Querschnittsgeometrien denkbar, beispielsweise rechteckige Querschnitte. In diesem Fall ist es auch vorstellbar, die Nuten 15,16 mit einem rechteckigen Querschnitt auszuführen.

Durch das Auffädeln des Implantats 17 auf den inneren Stützkörper 9 wird automatisch das schlauchförmige Element 1 über den Ausrichtabschnitt 27 gezogen, so dass es diesen umhüllt. Jener Abschnitt des schlauchförmigen Elementes 1, der über den Ausrichtabschnitt 27 hinausragt, also nicht mehr aufgefädelt werden kann, wird zunächst in sich eingestülpt (invaginiert). Danach kann dieser invaginierte Abschnitt entweder vor dem Ausrichtabschnitt 27 belassen werden oder aber in das Aufnahmevolumen 27a gestopft und dort vorzugsweise gefaltet oder auf andere Art komprimiert untergebracht werden.

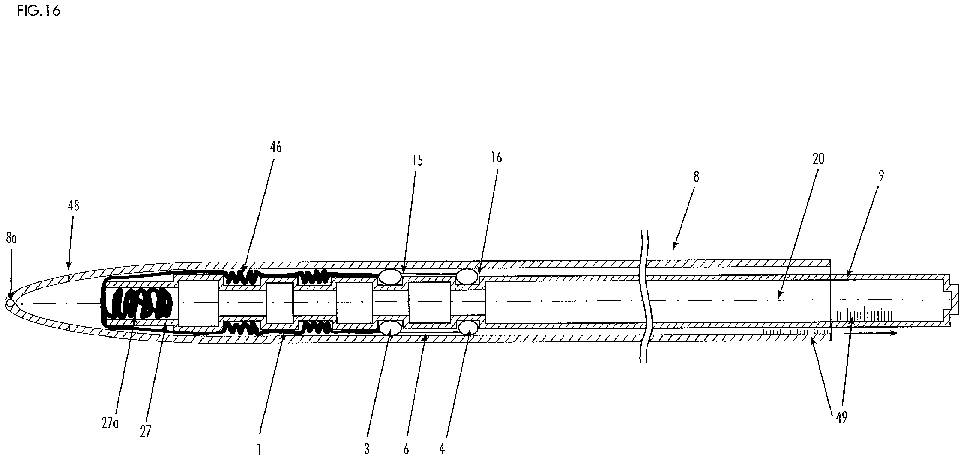

Zusätzlich kann es vorgesehen sein, an der Oberfläche des inneren Stützkörpers 9 Ausnehmungen 46 vorzusehen, in welche Abschnitte des schlauchförmigen Elementes 1 gerafft untergebracht werden können. Derartige Ausnehmungen 46 sind rein beispielhaft in Fig.16 dargestellt und dienen als Zugentlastung für den Fall, dass das schlauchförmige Element 1 vor der Fixierung des gastrointestinalen Implantats 17 mittels der Befestigungsmittel 3,4 am Pylorus 7 entfaltet wird, da in diesem Fall kein Zug vom schlauchförmigen Element 1 auf die Befestigungsmittel 3,4 ausgeübt werden kann, der die Expansion der Befestigungsmittel 3,4 negativ beeinflusst.

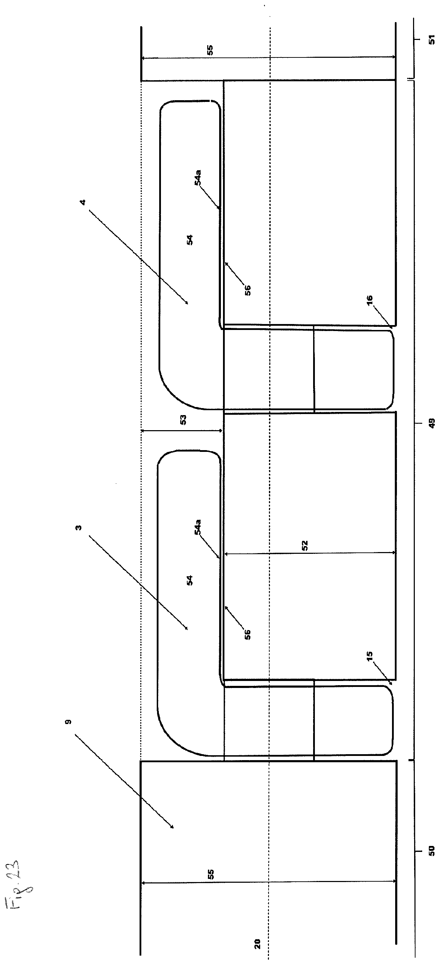

Eine besonders bevorzugte Ausführungsform des inneren Stützkörpers 9 ist in Fig.23 dargestellt. Ein Abschnitt 49 des inneren Stützkörpers 9 ist dabei mit einer gegenüber an diesen angrenzenden Bereichen 50,51 reduzierten Querschnittsfläche 52 ausgebildet. Die Nuten 15,16 sind in diesem Abschnitt 49 angeordnet. Die fehlende Querschnittsfläche 53 ermöglicht die Unterbringung von Abschnitten 54 der Befestigungselemente 3,4 in diesem Bereich, dh. jeweils ein Abschnitt 54 der Befestigungselemente 3,4 wird in Richtung der Längsachse 20 umgeklappt, wie dies in Fig.23 dargestellt ist. Das aufstellen der Abschnitte 54 wird durch das in dieser Figur nicht dargestellte äußere Behältnis 8 verhindert.

Bei entsprechender Dimensionierung der reduzierten Querschnittsfläche 52 im Verhältnis zur Querschnittsfläche 55 der angrenzenden Bereiche 50,51 und der Befestigungselemente 3,4 sowie der Nuten 15,16 können die Befestigungselemente 3,4 so gefaltet werden, dass diese vollkommen innerhalb der Querschnittsfläche 55 der angrenzenden Bereiche 50,51 angeordnet sind und nicht über diese hinausragen, wodurch eine besonders starke Komprimierung möglich ist.

Wie bereits weiter oben ausgeführt weist der innere Stützkörper 9 einen kreisförmigen Querschnitt auf, dh. die Querschnittsflächen 55 sind bevorzugt kreisförmig. Die reduzierte Querschnittsfläche 52 kann grundsätzlich beliebige Form aufweisen, ist aber bevorzugt kreissektorförmig ausgebildet, kann aber auch durch Entfernung einer oder zwei vorzugsweise einander gegenüberliegender, kreissegmentförmiger Querschnittsfläche(n) aus einer kreisförmigen Querschnittsfläche entstehen. In letzterem Fall bildet sich jedenfalls zumindest eine sehr gute Auflagefläche 56 für die Abschnitte 54 bzw. deren Stirnflächen 54a aus.

Lediglich der Vollständigkeit wegen ist anzumerken, dass die Querschnittsflächen 55 der Abschnitte 50,51 auch unterschiedlich groß sein können.

### FUNKTIONSWEISE DER ERFINDUNG

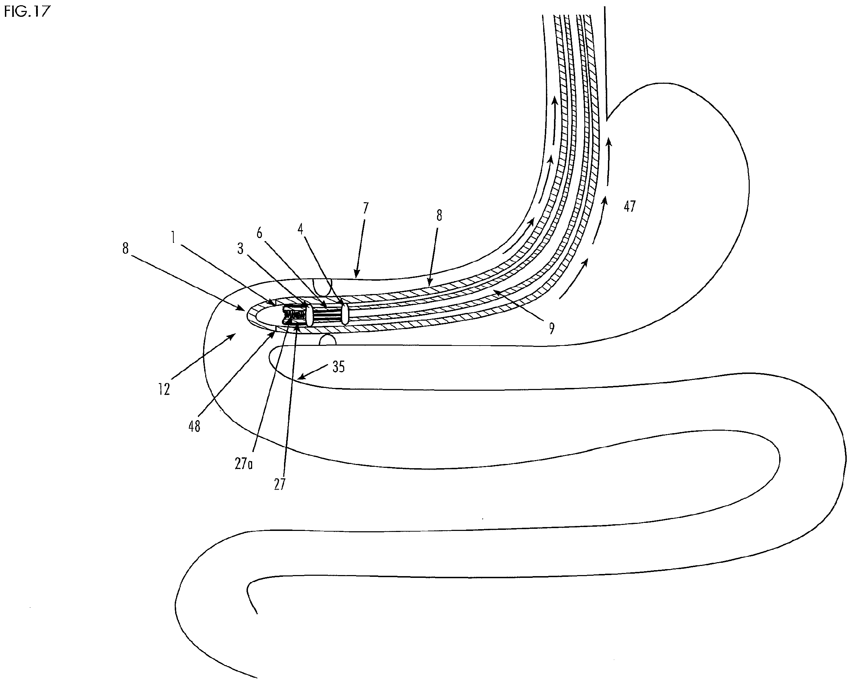

Im Anschluss erfolgt nun eine detaillierte Beschreibung der Funktionsweise der Erfindung anhand der Figuren 17 bis 21, welche diese lediglich schematisch darstellen.

Zunächst wird die Positioniervorrichtung 18, wie sie in den Fig. 12 oder 13 beispielhaft mit appliziertem gastrointestinalen Implantat 17 dargestellt ist, transoral, dh. über den Mund und die Speiseröhre in den Magen-/Darmtrakt eines Patienten eingeführt bis das erste Befestigungsmittel 3 eine Position eingenommen hat, die distal des Pylorus 7 liegt und das zweite Befestigungsmittel 4 ein Position, die proximal des Pylorus liegt. Dies geschieht mittels eines Führungsdrahtes (nicht gezeichnet) der zunächst mittels eines Gastroskops entsprechend in den Magen-/Darmtrakt eingebracht wird. Danach wird das äußere Behältnis 8 über den Führungsdraht an der vorgesehen Position im Magen-/Darmtrakt positioniert und der Führungsdraht wieder entgegen der Einführrichtung entfernt.

Zur Bestimmung der Position der Positioniervorrichtung 18 ist es vorteilhaft, wenn das äußere Behältnis 8 und/oder der innere Stützkörper 9, an deren Außenseiten mit einer Markierung 49 versehen sind, die dem den Eingriff vornehmenden Arzt die Einführtiefe des äußeren Behältnisses 8 anzeigt sowie die relative Position des inneren Stützkörpers 9 hierzu, so dass exakt festgestellt werden kann, wenn die Befestigungsmittel 3,4 ihre Position distal und proximal des Pylorus 7 erreicht haben. So kann beispielsweise die Distanz zwischen der Zahnreihe des Patienten und des Pylorus vorab gastroskopisch ausgemessen werden.

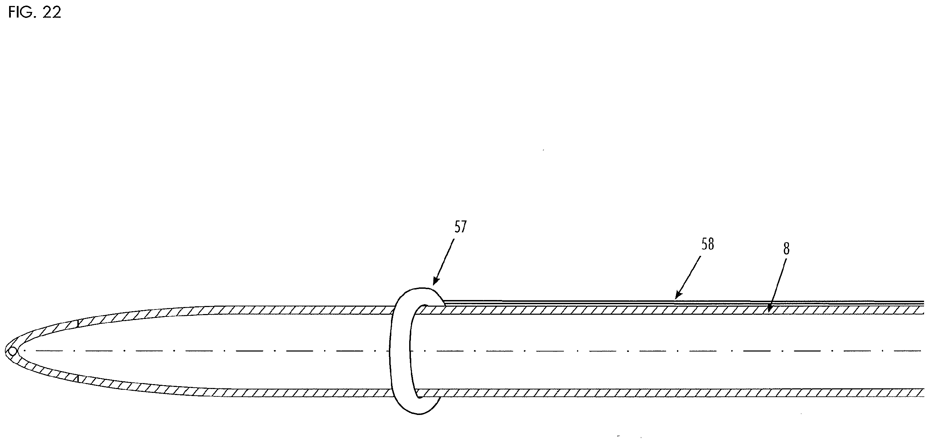

Alternativ oder zusätzlich können Anschlagmittel 57 (Fig.22) vorgesehen sein, welche das Einschieben des äußeren Behältnisses 8 bis zu einer definierten Position erleichtern. Das Anschlagmittel 57 kann beispielsweise als ringförmiger Ballon ausgebildet sein, der fix mit dem äußeren Behältnis 8 verbunden ist und dieses umgibt und über einen Insufflationsschlauch 58, der an der Oberfläche des äußeren Behältnisses geführt ist, mit Luft gefüllt werden kann. Befindet sich der Ballon im Magen, so wird dieser aufgeblasen, so dass die Positioniervorrichtung 18 lediglich so weit vorgeschoben werden kann, bis der Ballon am Pylorus anstößt. Die Lage des Ballons am äußeren Behältnis ist dabei so gewählt, dass sich zu diesem Zeitpunkt das erste Befestigungsmittel 3 in einer Lage befindet, in welcher dessen Expansion vorgenommen werden kann.

Wie bereits eingangs erwähnt, ist es vorteilhaft, wenn zu diesem Zeitpunkt das äußere Behältnis 8 und der innere Stützkörper 9 über Fixiermittel (nicht gezeichnet) zueinander lagefixiert sind.

Die folgenden Ausführungen sind allerdings unabhängig davon zu sehen, wie die Positioniervorrichtung 18 in die korrekte Position gebracht wurde.

Fig.17 zeigt jenen Zeitpunkt nach dem transoralen Einführen, zu welchem sich das erste Befestigungsmittel 3 distal des Pylorus 7 befindet. Zu diesem Zeitpunkt ist das distale Ende 12 des äußeren Behältnisses 8 noch verschlossen. Der distale Endbereich 1b des schlauchförmigen Elementes 1, der sich in einem mehr oder weniger eingestülpten Zustand im Aufnahmevolumen 27a des Ausrichtabschnitts 27 befindet kann ebenfalls verschlossen oder aber auch offen sein.

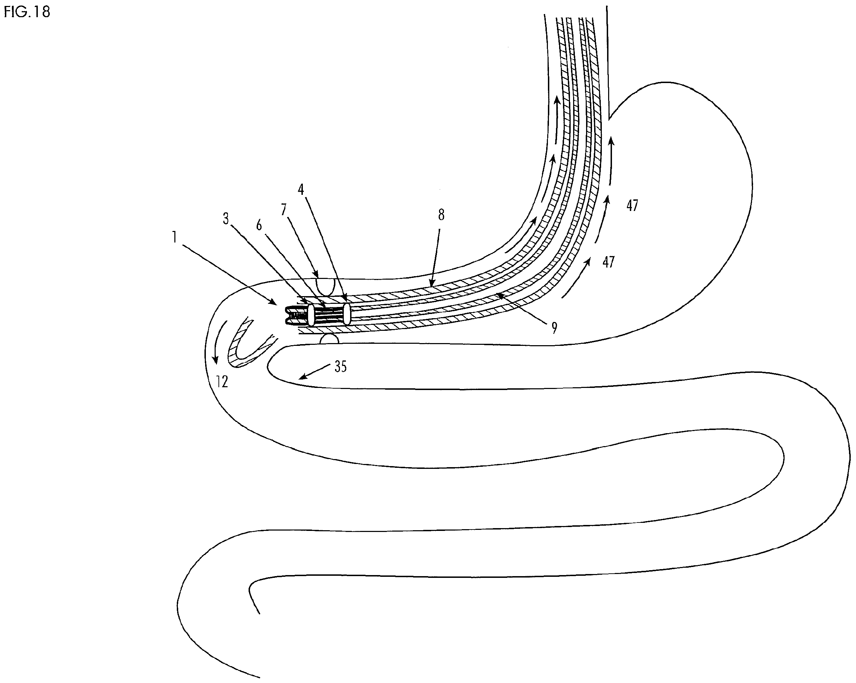

In weiterer Folge wird durch das geringfügige Zurückziehen des äußeren Behältnisses 8 in Richtung der Pfeile 47 und dem damit verbundenen Anstoßen des Ausrichtabschnitts 27 am distalen Endbereich 12, dieser vom restlichen äußeren Behältnis 8 abgetrennt, wie dies in Fig.18 gezeigt ist, oder aber lediglich geöffnet, je nachdem, ob die dies ermöglichende Strukturschwächung 48 über den gesamten Umfang des äußeren Behältnisses 8 verläuft oder nur einem Abschnitt des Umfangs. Alternativ, für den Fall, dass der distale Endbereich 12 aus resorbierbarem Material aufgebaut ist, kann auch dessen Auflösung abgewartet werden.

Beim Zurückziehen des äußeren Behältnisses 8 ist jedenfalls zunächst darauf zu achten, dass dieses nach dem Abtrennen oder Öffnen des distalen Endbereichs 12 nicht über die Position des ersten Befestigungsmittels 3 zurückgezogen wird.

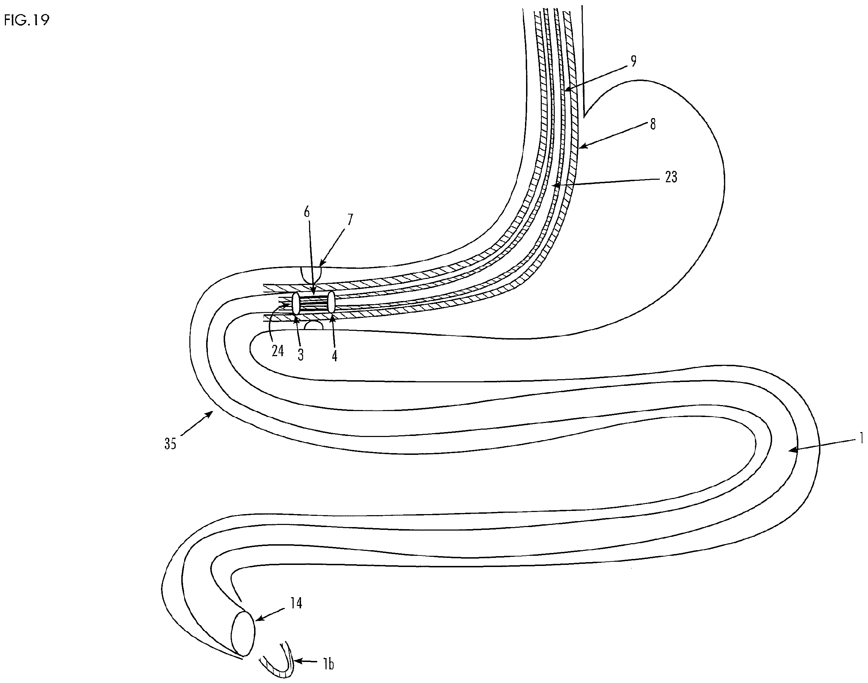

In weiterer Folge und wie in Fig. 19 gezeigt, kann über einen im inneren des Stützkörpers 9 verlaufenden Kanal 23, der in einer im Aufnahmevolumen 27a ragenden Auslassöffnung 24 endet (siehe auch Fig.9 und 10) ein Fluid, vorzugsweise CO₂ oder Luft über das offene Ende 1a des schlauchförmigen Elementes 1 kontinuierlich oder pulsativ in diesen eingeblasen werden. Durch den dadurch steigenden Innendruck wird der sich im Aufnahmevolumen 27a befindliche Abschnitt des schlauchförmigen Elementes 1 aus diesem herausbefördert und in Analogie zu einem Luftballon bis zu seiner gänzlichen Entfaltung aufgeblasen.

Soferne das distale Ende 1b des schlauchförmigen Elementes 1 geschlossen ist, muss dieses vom restlichen schlauchförmigen Element 1 gelöst werden, um dieses einsatzbereit zu machen. Dabei kann es vorgesehen sein, das distale Ende 1b aus resorbierbarem Material zu fertigen, so dass der die Intervention durchführende Arzt lediglich eine definierte Zeitspanne abwarten muss.

Alternativ dazu kann das schlauchförmige Element 1 in seinem Endbereich 1b mit einer Öffnung (nicht gezeichnet) versehen sein, die mit einem Stopfen (nicht gezeichnet) verschlossen ist oder mit einer Strukturschwächung 14 (siehe Fig.1), beispielsweise in Form einer Perforation oder eines verdünnten Wandabschnittes, die den zweiten Endbereich 1b vom restlichen schlauchförmigen Element 1 trennt. Durch das weitere Einblasen von Fluid in das zu diesem Zeitpunkt bereits vollständig entfaltete schlauchförmige Element 1 steigt dessen Innendruck weiter an, wodurch sich der distale Endbereich 1b vom restlichen schlauchförmigen Element 1 an der Strukturschwächung löst und auf natürlichem Weg ausgeschieden werden kann. Gleiches gilt für die Ausführungsform mit Stopfen, der beispielsweise aus Stärke geformt sein kann und sich ebenfalls durch den Innendruck aus der Öffnung löst, so dass das schlauchförmige Element nun für den Speisebrei durchgängig ist.

Für den Fall, dass das distale Ende des schlauchförmigen Elementes 1 nicht verschlossen ist, ist letzteres nach dem Entfalten aus dem Aufnahmevolumen 27a sofort einsatzbereit. Überraschenderweise hat sich herausgestellt, dass die Faltung oder Komprimierung des schlauchförmigen Elementes 1 im Aufnahmevolumen 27a ausreicht, um die Entfaltung durch das Einlasen des Fluids zu ermöglichen, auch wenn der distale Endbereich 1b nicht verschlossen ist.

In einer Ausführungsform der Erfindung ist es vorgesehen, dass im schlauchförmigen Element 1 ein Röntgenstreifen eingearbeitet ist, um die Lage des schlauchförmigen Elementes 1 bzw. dessen vollständige Entfaltung durch Röntgen sichtbar zu machen.

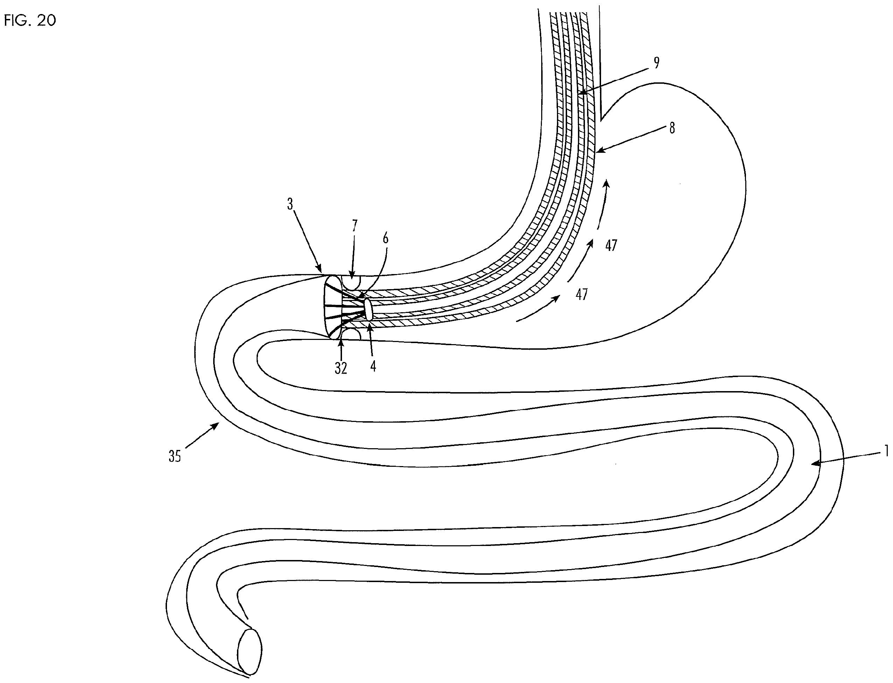

Als nächster Schritt kann nun das äußere Behältnis 8 über die Position des ersten Befestigungsmittels 3 in Richtung der Pfeile 47 zurückgezogen werden, wie dies in Fig.20 dargestellt ist, so dass sich das erste Befestigungsmittel 3 aus seiner Lagerstelle 3a lösen kann und es zur aboralen Expansion des ersten Befestigungsmittels 3 kommt. Zweckmäßigerweise ist der innere Stützkörper 9 mit Markierungen versehen, so dass leicht erkennbar ist, wie weit das äußere Behältnis 8 bereits zurückgezogen wurde und daraus wiederum geschlossen werden kann, ob und welches Befestigungsmittel 3,4 bereits expandieren konnte.

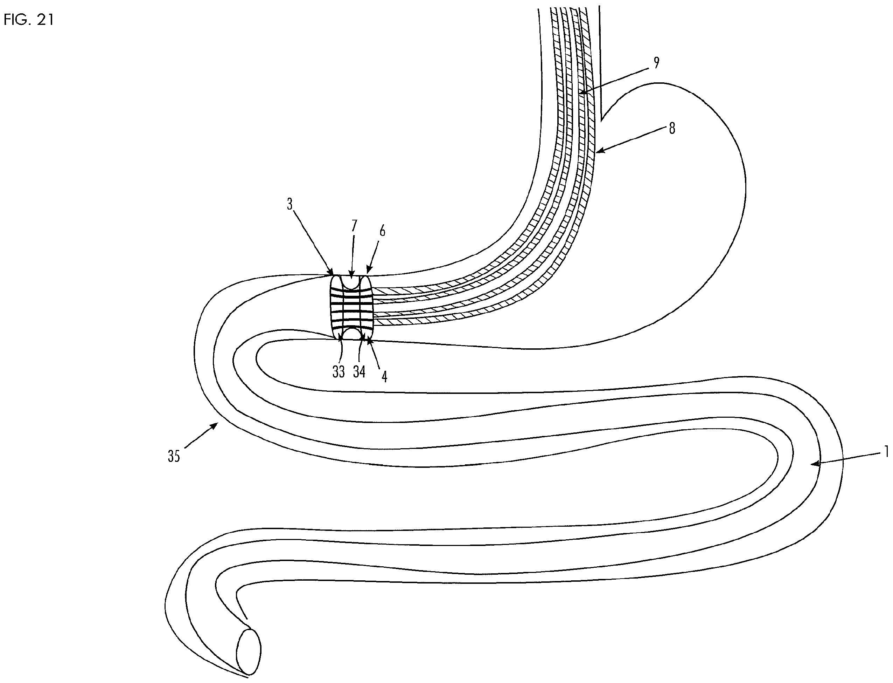

In der expandierten Form des ersten Befestigungsmittels 3 kann nun das äußere Behältnis 8 weiter in Richtung der Pfeile 47 zurückgezogen werden, so dass auch das zweite Befestigungsmittel 4 expandiert, wie dies in Fig.21 dargestellt ist. Zu diesem Zeitpunkt sind die beiden Befestigungsmittel 3,4 frei beweglich und werden durch das Verbindungselement 6 zueinander gezogen, so dass sie mit ihren Anlageflächen 33,34 an der distalen bzw. proximalen Stirnfläche 31,32 des Pylorus 7 anliegen und diesen klemmen. Die in den Ausnehmungen 46, sofern vorhanden, untergebrachten Schlauchreserven, unterstützen dieses Klemmen aufgrund der damit verbundenen, erleichterten Bewegungsmöglichkeit der Befestigungselemente 3,4 in axialer Richtung. Das gastrointestinale Implantat 17 ist damit lagefixiert, so dass in weiterer Folge sowohl das äußere Behältnis 8 als auch der innere Stützkörper 9 über die Speiseröhre wieder entfernt werden kann. Zurück bleibt das am Pylorus fixierte gastrointestinale Implantat 17 wie in Fig.2 dargestellt.

Es soll an dieser Stelle nicht unerwähnt bleiben, dass es auch möglich ist, das gastrointestinale Implantat 17 zuerst mittels der Befestigungsmittel 3,4 am Pylorus 7 zu fixieren und erst danach die Entfaltung des schlauchförmigen Elementes 1 durch Einblasen eines Fluids über den Kanal 23 zu bewirken.

### BEZUGSZEICHENLISTE

- 1.: schlauchförmiges Element
- 1a.: erstes (proximales) Ende des schlauchförmigen Elementes
- 1b.: zweites (distales) Ende des schlauchförmigen Elementes
- 2.: Zwölffingerdarm
- 3.: erstes Befestigungsmittel
- 3a.: erste Lagerstelle
- 4.: zweites Befestigungsmittel
- 4a.: zweite Lagerstelle
- 5.: Antrum des Magens
- 6.: Verbindungselement
- 7.: Pylorus
- 8.: äußeres Behältnis
- 8a.: Möglichkeit zur Aufnahme eines Führungsdrahtes
- 9.: innerer Stützkörper
- 10.: Durchflussöffnung des ersten Befestigungsmittels
- 11.: Durchflussöffnung des zweiten Befestigungsmittels
- 12.: distaler Endbereich des äußeren Behältnisses
- 13.: proximaler Endbereich des äußeren Behältnisses
- 14.: Strukturschwächung
- 15.: erste Nut
- 16.: zweite Nut
- 17.: Gastrointestinales Implantat
- 18.: Vorrichtung zur Positionierung eines gastrointestinalen Implantats im menschlichen Magen-Darm-Trakt
- 19.: Längsachse des äußeren Behältnisses
- 20.: Längsachse des inneren Stützkörpers
- 21.: rotationssymmetrischer Abschnitt des inneren Behältnisses
- 22.: ein Endbereich des inneren Stützkörpers
- 23.: Kanal im inneren Stützkörper
- 24.: Auslassöffnung Kanal
- 27.: Ausrichtabschnitt für schlauchförmiges Element
- 27a.: Aufnahmevolumen
- 29.: magnetischer Abschnitt des ersten Befestigungsmittel
- 29a.: Einzelmagnet eines magnetischen Abschnitts
- 30.: magnetischer Abschnitt des zweiten Befestigungsmittel
- 30a.: Einzelmagnet eines magnetischen Abschnitts
- 31.: proximale Stirnfläche des Pylorus
- 32.: distale Stirnfläche des Pylorus
- 33.: Anlagefläche des ersten Befestigungsmittels
- 34.: Anlagefläche des zweiten Befestigungsmittels
- 35.: Darm
- 36.: anziehende Magnetkraft
- 37.: abstoßende Magnetkraft
- 38.: Innenvolumen eines Stützelementes
- 40.: Spiralfederelement
- 41.: Stützelement
- 41a.: erster Endabschnitt des Stützelementes
- 41b.: zweiter Endabschnitt des Stützelementes
- 42.: Stützelement
- 43.: Spiralfederelement
- 44.: Spiralfederelement
- 45.: Spiralfederelement
- 46.: Ausnehmungen an der Oberfläche des inneren Stützkörpers
- 47.: Richtungspfeile
- 48.: Strukturschwächung des äußeren Behältnisses
- 49.: Abschnitt des inneren Stützkörpers mit reduzierter Querschnittsfläche
- 50.: an 49 angrenzender Abschnitt des inneren Stützkörpers
- 51.: an 49 angrenzender Abschnitt des inneren Stützkörpers
- 52.: reduzierte Querschnittsfläche
- 53.: fehlende Querschnittsfläche
- 54.: Abschnitt der Befestigungselemente 3,4
- 54a.: Stirnflächen der Abschnitte 54
- 55.: Querschnittsfläche der angrenzenden Abschnitte 51,52
- 56.: Auflagefläche
- 57.: Anschlagelement
- 58.: Insufflationsschlauch

## Patentansprüche

1. System umfassend ein gastrointestinales Implantat (17) mit einem ersten und zweiten Befestigungsmittel (3,4) sowie eine Vorrichtung (18) zur Positionierung desselben im menschlichen Magen-Darm-Trakt, wobei die Vorrichtung (18) wie folgt umfasst:
- ein eine erste Längsachse (19) aufweisendes äußeres Behältnis (8)
- einen im äußeren Behältnis angeordneten, eine zweite Längsachse (20) aufweisenden inneren Stützkörper (9)
- eine erste, zwischen äußerem Behältnis (8) und innerem Stützkörper (9) angeordnete Lagerstelle (3a) für das erste Befestigungsmittel (3)
- eine zweite, zwischen äußerem Behältnis (8) und innerem Stützkörper (9) angeordnete Lagerstelle (4a) für das zweite Befestigungsmittel (4), wobei
- das äußere Behältnis (8) vom inneren Stützkörper (9) entfernbar, vorzugsweise in Richtung der zweiten Längsachse (20) axial abziehbar ist
und das gastrointestinale Implantat (17) wie folgt umfasst:
- ein schlauchförmiges Element (1), ausgebildet um Speisebrei durch zumindest einen Abschnitt des menschlichen Darms zu befördern
- ein mit dem schlauchförmigen Element (1) verbundenes, eine Durchgangsöffnung (10) aufweisendes erstes Befestigungsmittel (3), ausgebildet, um in einem an den Pylorus angrenzenden Abschnitt des Zwölffingerdarms positioniert zu werden
- ein mit dem schlauchförmigen Element (1) oder mit dem ersten Befestigungsmittel (3) verbundenes, eine Durchgangsöffnung (11) aufweisendes zweites Befestigungsmittel (4), welches dazu ausgebildet ist, im Antrum (5) des Magens positioniert zu werden,
- ein Verbindungselement (6), welches das erste Befestigungsmittel (3) oder das schlauchförmige Element (1) mit dem zweiten Befestigungsmittel (4) verbindet, wobei erstes und zweites Befestigungsmittel (3,4) auf dem inneren Stützkörper (9) aufgefädelt sind und in der ersten bzw. zweiten Lagerstelle (3a,4a) lagefixiert und auf eine erste Größe komprimiert sind und/oder eine erste Position einnehmen, und bei Entfernung des äußeren Behältnisses (8) auf eine zweite Größe expandieren und/oder eine von der ersten Position unterschiedliche Position einnehmen, **dadurch gekennzeichnet, dass** wobei ein Abschnitt (49) des inneren Stützkörpers (9) mit einer gegenüber an diesen Abschnitt angrenzenden Bereichen (50,51) reduzierten Querschnittsfläche (52) ausgebildet ist, und dass in diesem Abschnitt die Lagerstellen (3a,4a) ausbildende Nuten (15,16) angeordnet sind, in welchen die beiden Befestigungsmittel (3,4) zumindest teilweise aufgenommen sind und jeweils ein Abschnitt (54) eines jeden Befestigungsmittels (3,4) in Richtung der Längsachse (20) des inneren Stützkörpers (9) umgeklappt ist.

2. System nach Anspruch 1, **dadurch gekennzeichnet, dass** der Mindestabstand der Nuten (15,16) zueinander zwischen 3mm und 70mm, vorzugsweise zwischen 5mm und 30mm beträgt.

3. System nach einem der vorhergehenden Ansprüche, **dadurch gekennzeichnet, dass** die gefalteten Befestigungsmittel (3,4) innerhalb der Querschnittsfläche (55) der angrenzenden Bereiche (50,51) angeordnet sind.

4. System nach einem der vorhergehenden Ansprüche, **dadurch gekennzeichnet, dass** die reduzierte Querschnittsfläche (52) des Abschnitts (4) des inneren Stützkörpers (9) eine Form aufweist, die durch Entfernung einer oder zweier vorzugsweise einander gegenüberliegender, kreissegmentförmiger Querschnittsflächen aus einer kreisförmigen Querschnittsfläche entsteht.

5. System nach Anspruch 4, **dadurch gekennzeichnet, dass** Stirnflächen (54a) der umgeklappten Abschnitte (54) eines jeden Befestigungsmittels (3,4) auf einer sich durch die Entfernung einer oder zweier vorzugsweise einander gegenüberliegender, kreissegmentförmiger Querschnittsflächen aus einer kreisförmigen Querschnittsfläche ergebenden Auflagefläche (56) aufliegen.

6. System nach einem der vorhergehenden Ansprüche, **dadurch gekennzeichnet, dass** das Verbindungselement (6) so ausgebildet ist, dass erstes und zweites Befestigungsmittel (3,4) in deren Position im Zwölffingerdarm (2) bzw. im Antrum (5) des Magens mit deren erster (33) bzw. zweiter (34) Anlagefläche unter Zwischenlage des Pylorus (7) gegeneinander gepresst werden, ohne dass es zu einem Abheben der Befestigungsmittel (3,4) vom Pylorus kommt und vorzugsweise aus Silikon gefertigt ist.

7. System nach einem der vorhergehenden Ansprüche, **dadurch gekennzeichnet, dass** das Verbindungselement (6) mehrere Bänder (6a) umfasst.

8. System nach einem der vorhergehenden Ansprüche, **dadurch gekennzeichnet, dass** das äußere Behältnis (8) hülsenförmig ausgebildet ist und ein distaler Endbereich (13) des äußeren Behältnisses (13) geschlossen und entfernbar oder öffenbar ausgebildet ist.

9. System nach einem der vorhergehenden Ansprüche, **dadurch gekennzeichnet, dass** das schlauchförmige Element (1) einen zweiten Endbereich (1b) aufweist, der geschlossen und entfernbar ausgebildet ist und vorzugsweise aus einem im Darm resorbierbaren Material gefertigt ist.

10. System nach einem der vorhergehenden Ansprüche, **dadurch gekennzeichnet, dass** ein dem distalen Endbereich (13) des äußeren Behältnisses (8) näher liegender Endbereich (22) des inneren Stützkörpers (9) einen Ausrichtabschnitt (27) für einen Abschnitt des schlauchförmigen Elementes (1) aufweist, welcher Ausrichtabschnitt (27) ein vorzugsweise becherförmiges Aufnahmevolumen (27a) aufweist.

11. System nach Anspruch 10, **dadurch gekennzeichnet, dass** das schlauchförmige Element (1) einen ersten Endbereich (1a) aufweist, der dem vorzugsweise becherförmig ausgebildeten Ausrichtabschnitt (27) übergestülpt ist.

12. System nach einem der Ansprüche 10 oder 11, **dadurch gekennzeichnet, dass** der nicht dem Ausrichtabschnitt (27) übergestülpte Abschnitt des schlauchförmigen Elementes (1) zumindest abschnittsweise, vorzugsweise zur Gänze in das Aufnahmevolumen (27a) eingestülpt ist und vorzugsweise darin gefaltet angeordnet ist.

## Claims

1. System comprising a gastrointestinal implant (17) with a first and a second fastening means (3, 4) and an apparatus (18) for positioning said system in the human gastrointestinal tract, wherein the apparatus (18) comprises the following:
- an outer vessel (8) having a first longitudinal axis (19)
- an inner supporting body (9) arranged inside the outer vessel and having a second longitudinal axis (20)
- a first mounting position (3a) arranged between outer vessel (8) and inner supporting body (9) for the first fastening means (3)
- a second mounting position (4a) arranged between outer vessel (8) and inner supporting body (9) for the second fastening means (4), wherein
- the outer vessel (8) is separable from the inner supporting body (9), can preferably be withdrawn axially in the direction of the second longitudinal axis (20) and the gastrointestinal implant (17) comprises as follows:
- a tubular element (1), embodied to transport chyme through at least one section of a human intestine;
- a first fastener (3), which is connected to the tubular element (1) and has a flow opening (10), the first fastener being designed to be positioned in a section of a duodenum adjacent to a pylorus
- a second fastener (4), which is connected to the tubular element (1) or to the first fastener (3) and has a flow opening (11), the second fastener being designed to be positioned in the pyloric antrum (5),
- a connecting element (6) that connects the first fastener (3) or the tubular element (1) to the second fastener (4), wherein
first and second fastening means (3, 4) are threaded on the inner supporting body (9) and are position-fixed in the first and second mounting positions (3a, 4a) respectively and are compressed to a first size and/or assume a first position, and upon removal of the outer vessel (8) expand to a second size and/or assume a second position that is different from the first position, **characterized in that** a section (49) of the inner supporting body (9) is constructed with a reduced cross-sectional area (52) compared with sections (50, 51) adjacent to this section, and that grooves (15, 16) that form the mounting positions (3a, 4a) are arranged in this section, in which grooves the two fastening means (3, 4) are at least partially accommodated and one section (54) of each of the fastening means (3, 4) is folded in the direction of the longitudinal axis (20) of the inner supporting body (9).

2. System according to Claim 1, **characterized in that** the minimum distance between the grooves (15, 16) is between 3 mm and 70 mm, preferably between 5 mm and 30 mm.

3. System according to any of the preceding claims, **characterized in that** the folded fastening means (3, 4) are arranged within the cross-sectional area (55) of the adjacent sections (50, 51) .

4. System according to any of the preceding claims, **characterized in that** the reduced cross-sectional area (52) of the section (4) of the inner supporting body (9) has a shape which upon the removal of one or two, preferably opposing circle segment-shaped cross-sectional surfaces forms a circular cross-sectional area.

5. System according to Claim 4, **characterized in that** faces (54a) of the folded back sections (54) of each fastening means (3, 4) bear on a support surface (56) created by the removal of one or two preferably opposing circle segment-shaped cross-sectional surfaces from a circular cross-sectional area.

6. System according to any of the preceding claims, **characterized in that** the connecting element (6) is designed such that first and second fastening means (3, 4) in their respective positions in the duodenum (2) and the pyloric antrum (5) are pressed against one another with their respective first (33) and second (34) contact surfaces with the pylorus (7) located therebetween, without resulting in a lifting off of the fastening means (3, 4) from the pylorus, and is preferably made of silicone.

7. System according to any of the preceding claims, **characterized in that** the connecting element (6) comprises multiple bands (6a) .

8. System according to any of the preceding claims, **characterized in that** the outer vessel (8) is designed in the form of a sleeve, and a distal end region (13) of the outer vessel (13) is designed to be closed and removable or openable.

9. System according to any of the preceding claims, **characterized in that** the tubular element (1) has a second end region (1b), the construction of which is closed and removable, and which is preferably made of a material that can be absorbed in the intestine.

10. System according to any of the preceding claims, **characterized in that** an end region (22) of the inner supporting body (9) located closer to the distal end region (13) of the outer vessel (8) has an aligning section (27) for a portion of the tubular element (1), which aligning section (27) includes a preferably cup-shaped holding volume (27a).

11. System according to Claim 10, **characterized in that** the tubular element (1) has a first end region (1a) that is imposed on the preferably cup-shaped aligning section (27).

12. System according to Claim 10 or 11, **characterized in that** the portion of the tubular element (1) that is not imposed on the aligning section (27) is at least partially, preferably entirely invaginated into the holding volume (27a) and preferably arranged in folded manner therein.

## Revendications

1. Système comprenant un implant gastro-intestinal (17) comportant un premier et un second moyen de fixation (3, 4) et un dispositif (18) de positionnement de celui-ci dans le tractus gastro-intestinal humain, le dispositif (18) comprenant les éléments suivants :
- un récipient extérieur (8) présentant un premier axe longitudinal (19),
- un corps support (9) intérieur présentant un second axe longitudinal (20) disposé dans le récipient extérieur,
- un premier point d'appui (3a) disposé entre le récipient extérieur (8) et le corps support intérieur (9) pour le premier moyen de fixation (3),
- un second point d'appui (4a) disposé entre le récipient extérieur (8) et le corps support intérieur (9) pour le second moyen de fixation (4),
- le récipient extérieur (8) pouvant être éloigné du corps support intérieur (9), de préférence extrait axialement en direction du second axe longitudinal (20),
et l'implant gastro-intestinal (17) comprenant les éléments suivants :
- un élément de forme tubulaire (1), conçu pour transporter du chyme à travers au moins une section de l'intestin humain,
- un premier moyen de fixation (3) connecté à l'élément de forme tubulaire (1), présentant un orifice traversant (10), et conçu pour être positionné au niveau de la section jouxtant le pylore du duodénum,
- un second moyen de fixation (4) connecté à l'élément de forme tubulaire (1) ou au premier moyen de fixation (3) et présentant un orifice traversant (11), qui est conçu pour être positionné dans l'antre (5) de l'estomac,
- un élément de connexion (6) qui connecte le premier moyen de fixation (3) ou l'élément de forme tubulaire (1) au second élément de fixation (4),
le premier et second moyen de fixation (3, 4) étant enfilés sur le corps support intérieur (9) et ayant un appui fixe (3a, 4a) dans le premier ou second point d'appui et étant comprimés réduite pour le premier moyen de fixation (3) à une première taille et/ou prenant une première position et, en cas d'enlèvement du récipient extérieur (8), se dilatant jusqu'à une seconde taille et/ou prenant une position différente de la première position, **caractérisé en ce qu'**une section (49) du corps d'appui intérieur (9) est réalisée avec une surface de section transversale (52) réduite par rapport aux zones (50, 51) jouxtant cette section, et que, dans cette section, sont disposées les rainures (15, 16) constituant les points d'appui (3a, 4a), dans lesquelles les rainures les deux moyens de fixation (3, 4) sont reçus du moins partiellement et que respectivement une section (54) de chaque moyen de fixation (3, 4) est rabattue en direction de l'axe longitudinal (20) du corps support intérieur (9).

2. Système selon la revendication 1, **caractérisé en ce que** la distance minimale entre les rainures (15, 16) est de 3 mm à 70 mm, de préférence 5 mm à 30 mm.

3. Système selon une des revendications précédentes, **caractérisé en ce que** les moyens de fixation pliés (3, 4) sont disposés dans la surface de section transversale (55) des zones contiguës (50, 51).

4. Système selon une des revendications précédentes, **caractérisé en ce que** la surface de section transversale réduite (52) de la section (4) du corps d'appui intérieur (9) présente une forme qui est issue de l'enlèvement d'une ou préférence de deux surfaces de section transversale en forme d'arcs de cercle de opposées d'une surface de section transversale de forme circulaire.

5. Système selon la revendication 4, **caractérisé en ce que** les surfaces avant (54a) des sections rabattues (54) de chaque moyen de fixation (3, 4) reposent sur une surface d'appui (56) obtenue en enlevant une ou deux surfaces de section transversale opposées en forme d'arcs de cercle d'une surface de section transversale circulaire.

6. Système selon une des revendications précédentes, **caractérisé en ce que** l'élément de connexion (6) est réalisé de manière à ce que le premier et le second moyen de fixation (3, 4) soient comprimés l'un contre l'autre dans leur position dans le duodénum (2) ou dans l'antre (5) de l'estomac par leur première (33) ou seconde (34) surface d'appui avec interposition du pylore (7) sans fil qu'il y ait un relèvement des moyens de fixation (3, 4) du pylore et est de préférence fabriquée à partir de silicone.

7. Système selon une des revendications précédentes, **caractérisé en ce que** l'élément de connexion (6) comprend plusieurs bandes (6a) .

8. Système selon une des revendications précédentes, **caractérisé en ce que** le récipient extérieur (8) est réalisé en forme de tube et qu'une zone terminale distale (13) du récipient extérieur (13) est fermée et est réalisée de manière à pouvoir être enlevée ou ouverte.

9. Système selon une des revendications précédentes, **caractérisé en ce que** l'élément de forme tubulaire (1) présente une seconde zone terminale (1b) qui est réalisée de manière à être fermée et à pouvoir être enlevé et est de préférence fabriquée à partir d'un matériau résorbable dans l'intestin.

10. Système selon une des revendications précédentes, **caractérisé en ce qu'**une zone terminale (22) plus proche de la zone terminale distale (13) du récipient extérieur (8) du corps d'appui intérieur (9) présente une section d'alignement (27) qui présente un volume récepteur de préférence en forme de godet (27a) pour une section l'élément de forme tubulaire (1) .

11. Système selon la revendication 10, **caractérisé en ce que** l'élément de forme tubulaire (1) présente une première zone terminale (la) qui est emboîtée sur la section d'alignement (27), de préférence en forme de godet.

12. Système selon une des revendications 10 ou 11, **caractérisé en ce que** la section non emboîtée sur la section d'alignement (27) de l'élément de forme tubulaire (1) est emboîtée du moins par section, de préférence en totalité dans le volume récepteur (27a) et y est de préférence disposée pliée.
